# EUROPÄISCHE PATENTSCHRIFT

(11) **EP 2 021 111 B1**
(45) Veröffentlichungstag und Bekanntmachung des Hinweises auf die Patenterteilung: **09.03.2011**
(21) Anmeldenummer: 07725681.6
(22) Anmeldetag: 30.05.2007
(51) Int. Cl.: B01D 69/14

(54) **ANORDNUNG FÜR EINE BIOLOGISCH FUNKTIONELLE MEMBRAN, SENSORANORDNUNG, FILTERANORDNUNG SOWIE DEREN VERWENDUNGEN**
ARRANGEMENT FOR A BIOFUNCTIONAL MEMBRANE, SENSOR ARRANGEMENT, FILTER ARRANGEMENT AND THE USES THEREOF
AGENCEMENT DESTINÉ À UNE MEMBRANE BIOFONCTIONNELLE, DISPOSITIF DÉTECTEUR, DISPOSITIF DE FILTRATION ET LEURS UTILISATIONS

(30) Priorität: 31.05.2006 DE 102006025344
(43) Veröffentlichungstag der Anmeldung: 11.02.2009
(73) Patentinhaber: Max-Planck-Gesellschaft zur Förderung der Wissenschaften e.V., 80539 München (DE)
(72) Erfinder: KÜHLBRANDT, Werner, 60594 Frankfurt am Main (DE); YILDIZ, Özkan, 60435 Frankfurt am Main (DE)
(74) Vertreter: Müller - Hoffmann & Partner
(86) Internationale Anmeldenummer: PCT/EP2007/004792
(87) Internationale Veröffentlichungsnummer: WO 2007/137840

(56) Entgegenhaltungen:
- WO-A-2004/011600
- WO-A-2006/122566
- WO-A-2007/047498
- WO-A-2007/084103
- YILDIZ OZKAN ET AL: "Structure of the monomeric outer-membrane porin OmpG in the open and closed conformation." THE EMBO JOURNAL 9 AUG 2006, Bd. 25, Nr. 15, 9. August 2006 (2006-08-09), Seiten 3702-3713, XP002454011 ISSN: 0261-4189
- SUBBARAO ET AL: "Crystal Structure of the Monomeric Porin OmpG" JOURNAL OF MOLECULAR BIOLOGY, LONDON, GB, Bd. 360, Nr. 4, 21. Juli 2006 (2006-07-21), Seiten 750-759, XP005593287 ISSN: 0022-2836
- CONLAN SEAN ET AL: "Folding of a monomeric porin, OmpG, in detergent solution." BIOCHEMISTRY 12 AUG 2003, Bd. 42, Nr. 31, 12. August 2003 (2003-08-12), Seiten 9453-9465, XP002454012 ISSN: 0006-2960
- CHRISTOF M. NIEMEYER, A. MIRKIN (EDITORS): "Nanobiotechnology" [Online] 28. Januar 2005 (2005-01-28), WILEY , XP002454032 ISBN: 978 3 527 60245 2 Gefunden im Internet: URL:http://www3.interscience.wiley.com/cgi -bin/booktext/109871972/BOOKPDFSTART> [gefunden am 2007-10-05] chapter 7 "Engineered Nanopores", H. Bayley Seite 93 - Seite 111 Seite 107, letzter Absatz
- H. BAYLEY: "Genetically engineered pores for sensing metal ions" OFFICE OF NAVAL RESEARCH - FINAL REPORT, 19. April 2002 (2002-04-19), XP008084573
- BAYLEY HAGAN ET AL: "Functional engineered channels and pores (Review)." MOLECULAR MEMBRANE BIOLOGY 2004 JUL-AUG, Bd. 21, Nr. 4, Juli 2004 (2004-07), Seiten 209-220, XP002454013 ISSN: 0968-7688
- CONLAN S ET AL: "Biochemical and biophysical characterization of OmpG: A monomeric porin." BIOCHEMISTRY 3 OCT 2000, Bd. 39, Nr. 39, 3. Oktober 2000 (2000-10-03), Seiten 11845-11854, XP002454014 ISSN: 0006-2960 in der Anmeldung erwähnt
- DENISE WONG ET AL: "Single molecule measurements of channel proteins incorporated into biomimetic polymer membranes" NANOTECHNOLOGY, IOP, BRISTOL, GB, Bd. 17, Nr. 15, 14. August 2006 (2006-08-14), Seiten 3710-3717, XP020103932 ISSN: 0957-4484
- HILLER MATTHIAS ET AL: "Solid-state magic-angle spinning NMR of outer-membrane protein G from Escherichia coli." CHEMBIOCHEM : A EUROPEAN JOURNAL OF CHEMICAL BIOLOGY SEP 2005, Bd. 6, Nr. 9, September 2005 (2005-09), Seiten 1679-1684, XP002454015 ISSN: 1439-4227
- BEHLAU M ET AL: "Projection structure of the monomeric porin OmpG at 6 a resolution" JOURNAL OF MOLECULAR BIOLOGY, LONDON, GB, Bd. 305, Nr. 1, 5. Januar 2001 (2001-01-05), Seiten 71-77, XP004466157 ISSN: 0022-2836
- ASTIER ET AL: "Protein components for nanodevices" CURRENT OPINION IN CHEMICAL BIOLOGY, CURRENT BIOLOGY LTD, LONDON, GB, Bd. 9, Nr. 6, Dezember 2005 (2005-12), Seiten 576-584, XP005162591 ISSN: 1367-5931
- FAJARDO D A ET AL: "Biochemistry and regulation of a novel Escherichia coli K-12 porin protein, OmpG, which produces unusually large channels." JOURNAL OF BACTERIOLOGY SEP 1998, Bd. 180, Nr. 17, September 1998 (1998-09), Seiten 4452-4459, XP002454016 ISSN: 0021-9193
- BAYLEY H: "DESIGNED MEMBRANE CHANNELS AND PORES" CURRENT OPINION IN BIOTECHNOLOGY, LONDON, GB, Bd. 10, 1999, Seiten 94-103, XP001058234 ISSN: 0958-1669

## Beschreibung

Die vorliegenden Erfindung betrifft Anordnungen für eine biologisch funktionelle Membran sowie deren Verwendung.

Die vorliegende Erfindung betrifft insbesondere die Entwicklung von schaltbaren Biosensoren, Elektroden, Nanofiltern und selektiven Zellenmembrankanälen, insbesondere für biomedizinische Anwendungen, und zwar auf der Grundlage der Proteinstruktur, des Außenmembranporins G von Escherichia coli, welches auch outer membrane porin G oder OmpG genannt wird.

Bei der Entwicklung moderner Anordnungen für Sensoren und/oder von materiellen Filtern finden vermehrt Strukturen Anwendung, die in der einen oder anderen Weise natürliche Membranstrukturen nachahmen, indem eine Lipiddoppelschicht als Lipiddoppelschichtmembran bereitgestellt wird, in welche bestimmte Strukturen, gebildet von Aminosäuresequenzen, eingebettet werden, um funktionell aktiv zu sein und das zunächst bestehende impermeable Verhalten der Lipiddoppelschichtmembran als solcher in selektiver Art und Weise und/oder gesteuert aufzuheben.

In der Veröffentlichung "Folding of monomeric porine, OmpG, in detergent solution", Biochemistry 2003. Vol. 42. Seiten 9453 - 9465 werden an Lipiddoppelschichtmembranen OmpG aus E. coli sowie einige Mutanten davon untersucht.

Auch die Veröffentlichung "Engineered nanopores" in "Nanobiotechnology", 2005. herausgegeben von Niemeyer et al., diskutiert die Verwendung von monomerem Porin OmpG zur Bildung von Nanoporen.

Die Veröffentlichung "Biochemical and biophysical characterization of OmpG: A monomeric porine". Biochemistry 2000. 39. Seiten 11845 - 11854 untersucht rekombinante Formen des Porins OmpG aus E. coli aufgrund von Leitfähigkeitsuntersuchungen in Lipiddoppelschichten.

Der vorliegenden Erfindung liegt die Aufgabe zugrunde. Anordnungen für eine biologisch funktionelle Membran und in Folge für Sensoren und für materielle Filter anzugeben, welche auf besonders einfache, stabile und definierte Art und Weise eine selektive und/oder gesteuerte Aufhebung der Impermeabilität erlauben.

Gelöst wird die der Erfindung zugrunde liegende Aufgabe bei einer Anordnung für eine biologisch funktionelle Membran erfingdungsgemäß mit den Merkmalen eines der unabhängigen Patentansprüche 1 bis 3. Vorteilhafte Weiterbildungen der erfindungsgemäßen Anordnung für eine biologische funktionelle Membran sind jeweils Gegenstand der abhängigen Ansprüche. Ferner ist Gegenstand der vorliegenden Erfindung die Verwendung der erfindungsgemäßen Anordnung für eine biologisch funktionelle Membran gemäß Anspruch 9.

Kernidee der vorliegenden Erfindung in ihrem allgemeinsten Sinn ist für eine Anordnung für eine biologisch funktionelle Membran und in Folge für Anordnungen für Sensoren und für Anordnungen materieller Filter Lipiddoppelschichtmembranen als funktionelle Membranen vorzusehen, in welche eine Mehrzahl Porineinheiten oder deren Derivate membrandurchspannend und funktionell als Poren oder Kanäle vorgesehen sind. Das Vorsehen der Porineinheiten oder deren Derivate als Poren oder Kanäle ermöglicht das erfindungsgemäße besonders einfache, stabile und definierte Aufheben der Impermeabilität der zugrunde liegenden Lipiddoppelschichtmembran als solcher, um besonders vorteilhafte Eigenschaften auszubildender biologisch funktioneller Membranen. Sensoranordnungen oder Filteranordnungen bereitzustellen.

Gemäß einem ersten Aspekt der vorliegenden Erfindung wird eine Anordnung für eine biologisch funktionelle Membran geschaffen.

Dabei wird erfindungsgemäß eine Anordnung für eine biologisch funktionelle Membran vorgeschlagen, die eine Lipiddoppelschichtmembran aufweist, in welcher eine Mehrzahl Porineinheiten P und/oder Derivate von Porineinheiten P membrandurchspannend und funktionell als - insbesondere schaltbare - Poren oder als - insbesondere schaltbare - Kanäle vorgesehen ist.

Unter einer Porineinheit P wird im allgemeinen ein funktionelle Einheit verstanden, welche die Funktion des Porins P als Pore oder als Kanal realisieren kann. Dies kann die kleinstmögliche Einheit sein. Dies ist jedoch nicht zwingend. Es kann darunter z.B. ein einzelnes Molekül, also ein Monomer verstanden werden. Denkbar sind aber auch Kombinationen oder Aggregate aus identischen Einheiten oder auch aus verschiedenen Einheiten.

Unter einem. Derivat der Porineinheit kann jeweils verstanden werden eine mittels chemischer, biochemischer und/oder molekularbiologischer Verfahren abgewandelte Form einer zugrunde liegenden Porineinheit. Insbesondere sind dabei auch Mutanten eingeschlossen, seinen diese natürlicher Art oder durch zielgerichtete Mutagenese erzeugte. Es ist auch möglich die Abwandlungsverfahren iteriert und in beliebiger Kombination miteinander aufeinander folgend auf eine zugrunde liegende Porineinheit anzuwenden, um ein Derivat zu erzeugen. Die zugrunde liegende Porineinheit kann dabei also eine Wildtypporineinheit oder selbst wieder ein Derivat einer Porineinheit sein.

Unter einer funktionellen Membran soll eine Membran der beschriebenen Form und Struktur ganz allgemein verstanden werden. Diese kann vollständig nativer oder natürlicher Herkunft sein, z.B. kann es sich um ein Membranfragment handeln, das aus einer Membran eines lebenden Organismus - z.B. aus einem Bakterium oder einem Oozyten - isoliert wurde. Der zugrunde liegende Organismus kann ein Wildtyporganismus oder eine Mutante sein. Die funktionelle Membran kann aber auch teilweise oder vollständig künstlicher Natur und z.B. über ein Rekonstitutionsverfahren gewonnen sein. Grundlage dafür können auch Liposomem oder Vesikel sein. Denkbar ist, dass die funktionelle Membran als Membranfragment, also als Sheet, als Liposom, als Vesikel oder als Mizelle vorliegt. Auch aus der Langmuir-Blodgett-Technik abgeleitete Strukturen sind denkbar.

Weitere Aspekte von Ausführungsformen der erfindungsgemäßen Anordnung für eine biologisch funktionelle Membran sind nachfolgend beschrieben:

Eine Anordnung für eine biologisch funktionelle Membran kann eine Lipiddoppelschichtmembran aufweisen,
- bei welcher eine Mehrzahl Porineinheiten und/oder Derivate von Porineinheiten membrandurchspannend und funktionell als Poren oder als Kanäle ausgebildet ist.
- bei welcher als Porineinheit ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher eine Porineinheit ausgebildet ist, bei welcher die Reste His231 und His261 jeweils durch Aspartat oder durch Glutamat ersetzt sind.

Eine andere Anordnung für eine biologisch funktionelle Membran kann eine Lipiddoppelschichtmembran aufweisen.
- bei welcher eine Mehrzahl Porineinheiten und/oder Derivate von Porineinheiten membrandurchspannend und funktionell als Poren oder als Kanäle ausgebildet ist.
- bei welcher als Porineinheit ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher eine Porineinheit ausgebildet ist, bei welcher die Reste His231 und His261 durch ein Ionenpaar ersetzt sind.
- wobei der Rest His231 jeweils durch Aspartat oder durch Glutamat und der Rest His261 jeweils durch Arginin oder Lysin ersetzt sind oder umgekehrt, und
- wobei der Rest His261 jeweils durch Aspartat oder durch Glutamat und der Rest His231 jeweils durch Arginin oder Lysin ersetzt sind.

Eine andere Anordnung für eine biologisch funktionelle Membran kann eine Lipiddoppelschichtmembran aufweisen.
- bei welcher eine Mehrzahl Porineinheiten und/oder Derivate von Porineinheiten membrandurchspannend und funktionell als Poren oder als Kanäle ausgebildet ist.
- bei welcher als Porineinheit ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher eine Porineinheit ausgebildet ist, bei welcher die Reste His231 und His261 so ersetzt sind, dass ein Reodoxsensor ausgebildet ist, und zwar
- indem die Reste His231 und His261 jeweils durch Cystein ersetzt sind.

Eine andere Anordnung für eine biologisch funktionelle Membran kann eine Lipiddoppelschichtmembran aufweisen.
- bei welcher eine Mehrzahl Porineinheiten und/oder Derivate von Porineinheiten membrandurchspannend und funktionell als Poren oder als Kanäle ausgebildet ist.
- bei welcher als Porineinheit ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher eine Porineinheit ausgebildet ist, bei welcher zwischen dem Anfang der Schleife L6 und dem Ende der Schleife L7 der Aminosäuresequenz der Rest Glu227 und im Bereich der Aminosäuresequenz auf der Innenseite der Pore einer oder mehrere der Reste Phe269. Glu17 und Val19 so ersetzt sind, dass ein Reodoxsensor ausgebildet ist, und zwar
- indem diese Reste jeweils durch Cystein ersetzt sind.

Eine andere Anordnung für eine biologisch funktionelle Membran kann eine Lipiddoppelschichtmembran aufweisen.
- bei welcher eine Mehrzahl Porineinheiten und/oder Derivate von Porineinheiten membrandurchspannend und funktionell als Poren oder als Kanäle ausgebildet ist,
- bei welcher als Porineinheit ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher die Schleife L6 der Aminosäuresequenz entfernt ist oder
- bei welcher diejenigen Histidinreste der Aminosäuresequenz ersetzt sind, welche zum pH-Sensor der Porineinheit gehören.

Als Porineinheit P kann ein Außenmembranporin G aus dem Bakterium Escherichia coli oder eine Derivat eines Außenmembranporins G aus dem Bakterium Escherichia coli vorgesehen sein.

Als Porineinheit P kann aber auch zusätzlich oder alternativ ein zum Außenmembranporin G aus dem Bakterium Escherichia coli oder zu einem Derivat eines Außenmembranporins G aus dem Bakterium Escherichia coli gleichartiges oder homologes 14-stränglges monomeres Porin aus einem - gegebenenfalls anderen - Bakterium oder aus einem Archaebakterium vorgesehen sein.

Weitere Aspekte werden nachfolgend definiert:

Als Porineinheit P kann ein natives Außenmembranporin G aus Escherichia coli vorgesehen sein.

Es kann eine Porineinheit P vorgesehen sein, welche im neutralen Bereich eines Umgebungsmediums, z.B. eines Messmediums, insbesondere im Bereich eines pH-Werts oberhalb von etwa 6.5 und bis etwa 7.5, eine geöffnete Pore der funktionellen Membran bildet. Darüber hinaus kann die Porineinheit auch so gewählt sein, dass im basischen Bereich, also insbesondere oberhalb eines pH-Werts von etwa 7.5. eine geöffnete Pore der funktionellen Membran gebildet ist.

Es kann eine Porineinheit P vorgesehen sein, welche im sauren Bereich eines Umgebungsmediums, z.B. eines Messmediums, insbesondere im Bereich eines pH-Werts von etwa unter 6.0. vorzugsweise im Bereich von etwa 5.5. eine geschlossene Pore der funktionellen Membran bildet.

Es kann eine Porineinheit P vorgesehen sein, welche im neutralen Bereich eines Umgebungsmediums, z.B. eines Messmediums, insbesondere im Bereich eines pH-Werts oberhalb von etwa 6.5 und bis etwa 7.5. eine geschlossene Pore der funktionellen Membran bildet. Darüber hinaus kann die Porineinheit auch so gewählt sein, dass im basischen Bereich, also insbesondere oberhalb eines pH-Werts von etwa 7.5. eine geschlossene Pore der funktionellen Membran gebildet ist.

Es kann eine Porineinheit P vorgesehen sein, welche im sauren Bereich eines Umgebungsmediums. z.B. eines Messmediums, insbesondere im Bereich eines pH-Werts von etwa unter 6.0. vorzugsweise im Bereich von etwa 5.5. eine geöffnete Pore der funktionellen Membran bildet.

Es kann eine Porineinheit P vorgesehen ist, welche in einem vergleichsweise niedrigeren Konzentrationsbereich eines Umgebungsmediums, z.B. eines Messmediums. - insbesondere unterhalb eines Bereich von etwa 1 pmol/l bis etwa 1 µmol/l - eines oder mehrerer Ionen aus der Gruppe, die gebildet wird von Gd³⁺, Cr^{2+/3+}, Ca²⁺, Cu⁺. Hg²⁺. Zn²⁺, Cd²⁺ und Pb²⁺, eine geöffnete Pore oder einen geöffneten Kanal für die funktionelle Membran bildet.

Es kann eine Porineinheit P vorgesehen sein, welche in einem vergleichsweise höheren Konzentrationsbereich eines Umgebungsmediums, z.B. eines Messmediums, - insbesondere in einem Bereich von etwa 1 pmol/l bis etwa 1 µmol/l - eines oder mehrerer Ionen aus der Gruppe, die gebildet wird von Gd³⁺, Cr^{2+/9+}, Ca²⁺, Cu⁺, Hg²⁺. Zn²⁺, Cd²⁺ und Pb²⁺, eine geschlossene Pore oder einen geschlossenen Kanal der funktionellen Membran bildet.

Es kann eine Porineinheit P vorgesehen ist, welche in einem vergleichsweise niedrigeren Konzentrationsbereich eines Umgebungsmediums, z.B. eines Messmediums. - insbesondere unterhalb eines Bereich von etwa 1 pmol/l bis etwa 1 µmol/l - eines oder mehrerer Ionen aus der Gruppe, die gebildet wird von Gd³⁺, Cr^{2+/3}, Ca²⁺, Cu⁺, Hg², Zn²⁺, Cd²⁺ und Pb²⁺, eine geschlossene Pore für die funktionelle Membran bildet.

Es kann eine Porineinheit P vorgesehen sein, welche in einem vergleichsweise höheren Konzentrationsbereich eines Umgebungsmediums, z.B. eines Messmediums. - insbesondere in einem Bereich von etwa 1 pmol/l bis etwa 1 µmol/l - eines oder mehrerer Ionen aus der Gruppe, die gebildet wird von Gd³⁺, Cr^{2+/3+}, Ca²⁺, Cu⁺, Hg²⁺, Zn²⁺, Cd²⁺ und Pb²⁺, eine geöffnete Pore der funktionellen Membran bildet.

Es kann eine Porineinheit P vorgesehen sein, welche eine permanent offene Pore oder einen permanent offenen Kanal der funktionellen Membran bildet.

Es kann eine Porineinheit P vorgesehen sein, bei welcher in der Schleife L6 oder im Loop L6 der Aminosäuresequenz eine, mehrere oder sämtliche Histidinreste ersetzt sind.

Vorangehend und im Folgenden werden die Begriffe Loop und Schleife im Zusammenhang mit der Aminosäuresequenz und der sich ergebenden tertiären und quartären Struktur synonym verwendet.

Es kann eine Porineinheit P vorgesehen sein, bei welcher zwischen dem Anfang der Schleife L6 und dem Ende der Schleife L7 der Aminosäuresequenz eine, mehrere oder sämtliche Histidinreste ersetzt sind durch jeweils eine der weiteren 19 essentiellen Aminosäuren, vorzugsweise durch eine der Aminosäuren mit geladenen Seitenketten. z.B. Aspartat. Glutamat. Arginin und Lysin, und/oder auch durch Cystein und/oder durch eine der aromatischen Aminosäuren Phenylalanin. Tryptophan. Tyrosin und/oder durch eine der Aminosäure mit hydrophoben Seitenketten, z.B. Methionin, Leucin, Isoleucin, Valin und Alanin, und/oder durch eine der Aminosäuren Serin. Threonin. Asparagin. Glutamin. Prolin und Glycin.

Es kann eine Porineinheit P vorgesehen sein, bei welcher zwischen dem Anfang der Schleife L6 und dem Ende der Schleife L7 der Aminosäuresequenz die Reste His231 und His261 jeweils durch Aspartat oder durch Glutamat ersetzt sind.

Es kann eine Porineinheit P vorgesehen sein, bei welcher zwischen dem Anfang der Schleife L6 und dem Ende der Schleife L7 der Aminosäuresequenz die Reste His231 und His261 durch ein Ionenpaar ersetzt sind, wobei insbesondere der Rest His231 durch eine saure Gruppe - z.B. jeweils durch Aspartat oder durch Glutamat - und der Rest His261 durch eine basische Gruppe - z.B. jeweils durch Arginin oder Lysin ersetzt sind oder wobei umgekehrt insbesondere der Rest His261 durch eine saure Gruppe - z.B. jeweils durch Aspartat oder durch Glutamat - und der Rest His231 durch eine basische Gruppe - z.B. jeweils durch Arginin oder Lysin ersetzt sind.

Es kann eine Porineinheit P vorgesehen sein, bei welcher zwischen dem Anfang der Schleife L6 und dem Ende der Schleife L7 der Aminosäuresequenz die Reste His231 und His261 so ersetzt sind, dass ein Reodoxsensor ausgebildet ist, wobei insbesondere die Reste His231 und His261 jeweils durch Cystein ersetzt sind.

Es kann eine Porineinheit P vorgesehen sein, bei welcher zwischen dem Anfang der Schleife L6 und dem Ende der Schleife L7 der Aminosäuresequenz der Rest Glu227 und im Bereich der Aminosäuresequenz auf der Innenseite der Pore einer oder mehrere der Reste Phe269, Glu17 und Val19 so ersetzt sind, dass ein Reodoxsensor ausgebildet ist, wobei insbesondere die ersetzten Reste jeweils durch Cystein ersetzt sind.

Es kann eine Porineinheit P vorgesehen sein, bei welcher eine oder mehrere Bindungsstellen, insbesondere für divalente und/oder trivalente Kationen, ausgebildet sind.

Die Bindungsstellen für divalente und/oder trivalente Kationen können ausgebildet sein in einer oder in mehreren Regionen aus der Gruppe, die gebildet wird von der Schleife L1 der Aminosäuresequenz, der Schleife L2 der Aminosäuresequenz, der Schleife L6 der Aminosäuresequenz, der Schleife L7 der Aminosäuresequenz und geeigneten Regionen im Bereich der Innenwand der gebildeten oder der potentiellen Pore der Porineinheit.

Es kann eine Bindungsstelle für Zn²⁺ ausgebildet sein durch Ersetzen von Glu227 durch einen Histidin und von einem oder mehreren der Reste Phe269. Glu17 und Va119 jeweils durch Cystein.

Es kann eine Bindungsstelle für Zn²⁺ ausgebildet sein durch Ersetzen von Ile226 und Arg228 durch Histidin und von Thr64 und Phe66 durch Cystein.

Gemäß einem weiteren Aspekt der vorliegenden Erfindung wird eine Sensoranordnung geschaffen.

Es wird somit erfindungsgemäß eine Sensoranordnung vorgeschlagen, bei welcher eine funktionelle Membran mit einer Anordnung gemäß der vorliegenden Erfindung vorgesehen ist.

Es kann ferner eine erste elektrisch leitfähige Elektrodeneinrichtung vorgesehen sein, weiter eine zweite elektrisch leitfähige Elektrodeneinrichtung und ein wässriges Messmedium, in welchem die erste und die zweite Elektrodeneinrichtung voneinander räumlich getrennt angeordnet sind, wobei durch die funktionelle Membran die ersten und zweiten Elektrodeneinrichtungen im Bereich des wässrigen Messmediums steuerbar elektrisch voneinander isolierbar und steuerbar elektrisch miteinander verbindbar sind.

Durch die funktionelle Membran kann das Messmedium in ein erstes Kompartiment und in ein zweites und davon getrenntes zweites Kompartiment unterteilt sein.

Im ersten Kompartiment kann die erste Elektrodeneinrichtung zumindest zum Teil angeordnet sein.

Im zweiten Kompartiment die zweite Elektrodeneinrichtung zumindest zum Teil angeordnet ist und als Gegenelektrode fungiert.

Die funktionelle Membran kann zumindest zum Teil mit einer Seite zumindest an einem Teil der Oberfläche der ersten Elektrodeneinrichtung immobilisiert sein.

Die Immobilisierung der funktionellen Membran an der ersten Elektrodeneinrichtung kann über eine chemische Bindung ausgebildet sein.

Die Immobilisierung der funktionellen Membran an der ersten Elektrodeneinrichtung kann über in der Lipiddoppelschichtmembran vorgesehene funktionelle Gruppen, insbesondere über Thiogruppen vorgesehener Thiolipide oder Thioalkane ausgebildet sein.

Die erste Elektrodeneinrichtung kann mit oder aus einem Edelmetall gebildet sein.

Das Edelmetall kann ein Metall aus der Gruppe sein, die gebildet wird von Gold. Silber und Platin.

Die erste Elektrodeneinrichtung und die zweite Elektrodeneinrichtung können außerhalb des wässrigen Messmediums mit einem Messkreis verbunden sein.

Gemäß einem anderen Aspekt der vorliegenden Erfindung wird eine Filteranordnung geschaffen. Diese weist eine funktionelle Membran mit einer Anordnung gemäß der vorliegenden Erfindung auf.

Die Filteranordnung kann einen Stützkörper aufweisen, der eine Vorderseite, eine Rückseite und Poren umfasst, die ihrerseits von der Vorderseite zur Rückseite reichen und diese miteinander verbinden.

Die Poren können oder ein Teil kann der durch eine oder durch eine Mehrzahl funktioneller Membranen querschnittsmäßig abgedeckt, überspannt oder verschlossen sein. Dies geschieht bevorzugt durch jeweils genau eine funktionelle Membran pro Pore. Denkbar ist aber auch, dass mehrere funktionelle Membranen pro Pore vorliegen, z.B. in Form eines Stapels.

Der Stützkörper kann mit oder aus einem Material aus der Gruppe ausgebildet sein, die besteht aus anorganischen Materialien, keramischen Materialien und organischen Materialien.

Der Stützkörper kann als Milliporefilter ausgebildet sein.

Die Poren des Stützkörpers können einen Durchmesser im Bereich von etwa 0.2 µm bis etwa 25 µm aufweisen, vorzugsweise im Bereich von etwa 0,2 µm bis etwa 2 µm.

Gemäß einem weiteren Aspekt der vorliegenden Erfindung werden auch verschiedene Verwendungen der erfindungsgemäßen Anordnung für eine biologisch funktionelle Membran, der erfindungsgemäßen Sensoranordnung und der erfindungsgemäßen Filteranordnung vorgeschlagen.

Die erfindungsgemäße Anordnung für eine biologisch funktionelle Membran kann in einer Sensoranordnung, insbesondere in einer Biosensoranordnung verwendet werden.

Die erfindungsgemäße Anordnung für eine biologisch funktionelle Membran kann in einer Filteranordnung verwendet werden.

Die erfindungsgemäße Anordnung für eine biologisch funktionelle Membran kann verwendet werden zur, in einem Mittel zur oder in einem Verfahren zur In-vitro- oder In-vivo-Ausbildung einer oder mehrerer selektiver oder selektiv gesteuerter oder steuerbarer Permeabilitäten - insbesondere spezifisch - in Membranen eines oder mehrerer Zelltypen, insbesondere im menschlichen oder tierischen Organismus.

Die erfindungsgemäße Anordnung für eine biologisch funktionelle Membran kann verwendet werden zur, in einem Mittel zur oder in einem Verfahren zur Krebstherapie.

Dabei kann die funktionelle Membran über zielgerichtete Mutagenese und/oder in einem Expressionsverfahren ausgebildet werden.

Die funktionelle Membran kann über einen Oberflächenkontakt der Porineinheiten mit einer bestehenden Membran und/oder über einen Membranfusionsprozess ausgebildet werden.

Die funktionelle Membran kann auch über einen spezifischen Antikörper-Antigen-Kopplungs- und -Erkennungsprozess ausgebildet werden.

Die erfindungsgemäße Sensoranordnung kann als Biosensoranordnung verwendet werden oder auch als eine Anordnung einer selektiven Elektrode oder für eine selektive Elektrode.

Die erfindungsgemäße Filteranordnung kann als eine Anordnung eines materiellen Nanofilters oder für einen materiellen Nanofilter verwendet werden, also insbesondere in einem materiellen Filter mit einer Ausschlussgröße im Bereich bis etwa 2 nm.

Diese und weitere Aspekte der vorliegenden Erfindung werden anhand der nachstehenden Bemerkungen weiter erläutert:

Die vorliegende Erfindung betrifft unter anderem die Entwicklung von schaltbaren Biosensoren. Elektroden. Nanofiltern und selektiven Zellemembrankanälen für biomedizinische Anwendungen auf der Grundlage der Proteinstruktur des "Outer Membrane Porin G" (OmpG) von *Escherichia coli*.

### Stand der Forschung

Außenmembranporine (outer membrane porins = OMPs) sind Proteine, die in der Außenmembran gram-negativer Bakterien Poren bilden, die dem Stoffaustausch und der Nährstoffaufnahme dienen. Sie wirken als molekulare Filter für Zucker. Aminosäuren. Nukleotide und ionische Verbindungen und schützen die Zelle vor schädlichen Stoffen.

Das Außenmembranporin G (OmpG) von *Escherichia coli* wird im Bakterium exprimiert, wenn andere Porine ausgeschaltet sind (Misra & Benson, 1989). Aus Bakterien isoliertes, natives OmpG bildet in künstlichen Membranen ausgedehnte zweidimensionale Gitter (Behlau et al, 2001), in denen das Molekül als Monomer vorliegt. Das Protein bildet große, unspezifische Poren für Moleküle bis zu 900 kDa (Fajardo et al, 1998), die bei neutralem pH offen sind, und in Gegenwart von Gd³⁺-Ionen (Conlan et al, 2000) bzw. im sauren Bereich (<pH6) schließen (Conlan et al. 2003). Rekombinantes OmpG kann durch bakterielle Fermentation kostengünstig in großem Maßstab in Form von Einschlusskörpern hergestellt werden, die durch einfaches Aufbrechen der Zellen und Zentrifugation leicht und in hoher Reinheit zu isolieren sind. Die Einschlusskörper bestehen aus denaturiertem, aggregiertem Protein, das in einem von uns entwickelten Verfahren nahezu quantitativ renaturiert und damit in die funktionale Form überführt wird. Renaturiertes OmpG bildet in künstlichen Membranen ebenfalls ausgedehnte zweidimensionale Gitter, ist voll funktionsfähig (Conlan et al. 2003) und hat die gleiche Struktur wie das native Protein.

### Röntgenstruktur von OmpG in der offenen und geschlossenen Konformation

Die Röntgenstruktur von renaturiertem OmpG wurde von uns bestimmt und ist in dem beiliegenden Manuskript erstmals beschrieben. OmpG ist ein Zylinder aus einem 14-strängigen beta- oder β-Faltblatt, das die Transmembranpore bildet. Die Pore hat auf einer Gesamtlänge von ca. 5 nm einen mittleren Durchmesser von ca. 15 nm, mit einer Verengung auf ca. 1 nm am extrazellulären Poreneingang. Das Poreninnere ist vorwiegend mit langen, geladenen Seitenketten gesäumt, wobei Gruppen gleicher Ladung am extrazellulären Poreneingang einander gegenüber liegen. Kristalle im sauren pH-Bereich zeigen die geschlossene Form bei 2,7 Å Auflösung, Kristalle im neutralen Bereich zeigen die offene Form bei 2.3 Å Auflösung. Das Öffnen und Schließen der Pore geschieht durch Umfalten einer extrazellulären Peptidschleife (Loop 6. L6), wobei zwei in der Struktur nebeneinander liegende Histidinreste (His231 am Ende von L6 und His261 am Beginn der benachbarten Schleife L7) als pH-Sensor und Schalter fungieren, die sich im protonierten Zustand bei saurem pH abstoßen, was die Verlängerung der Schleife L6 von 10 auf 17 Aminosäurereste bewirkt und die Konformationsänderung auslöst.

### Aspekte der Erfindung

Die Erfindung besteht unter anderem auch darin, die den Erfindern erstmals beschriebene Struktur von OmpG aus Escherichia coli und insbesondere den pH-Schalter zur Entwicklung von schaltbaren Biosensoren. Nanofiltern und selektiven Elektroden für (nano)technologische Anwendungen nutzbar zu machen.

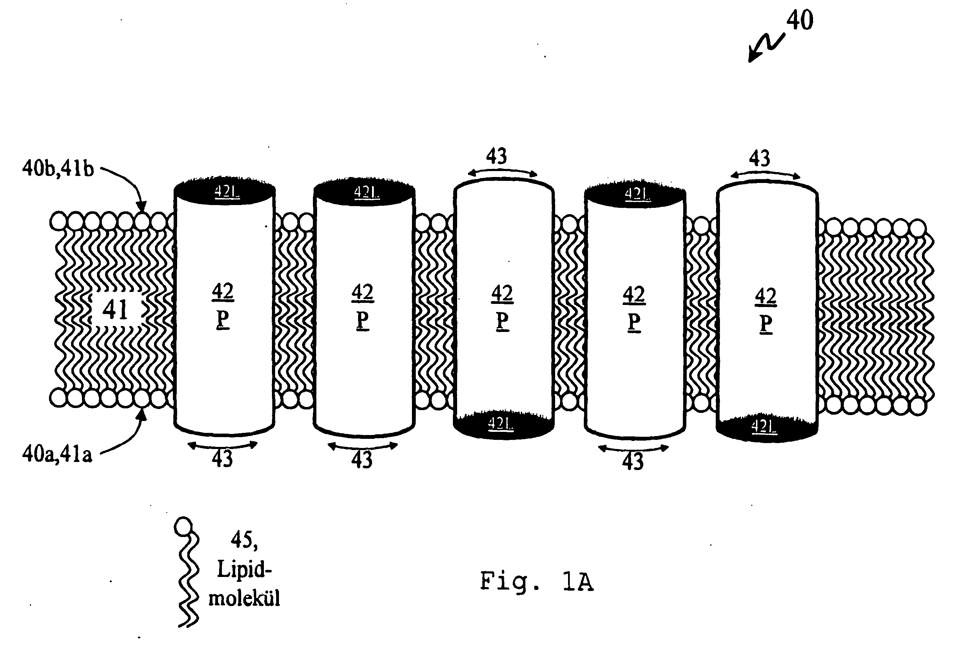

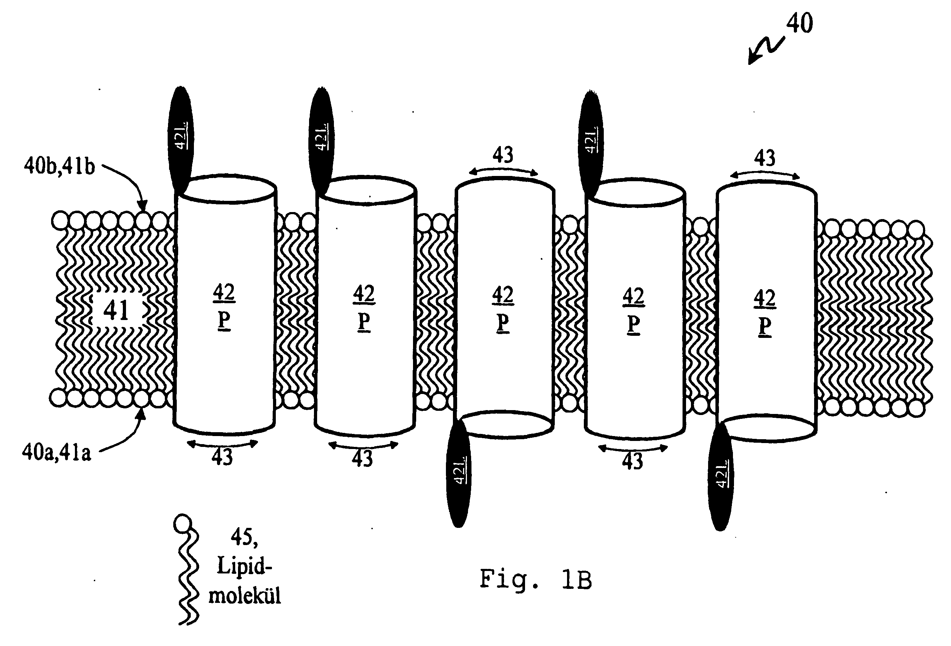

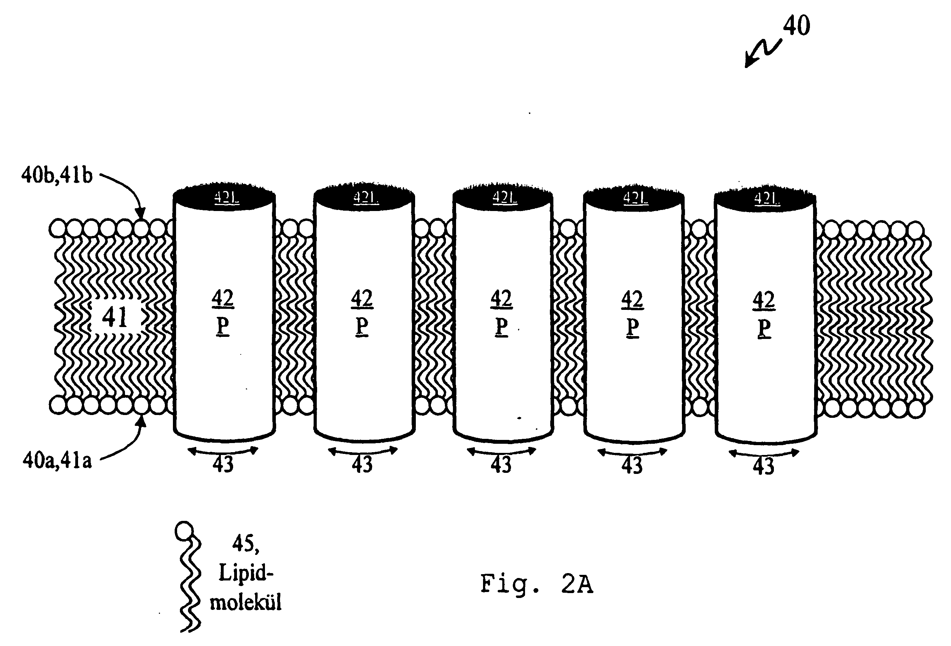

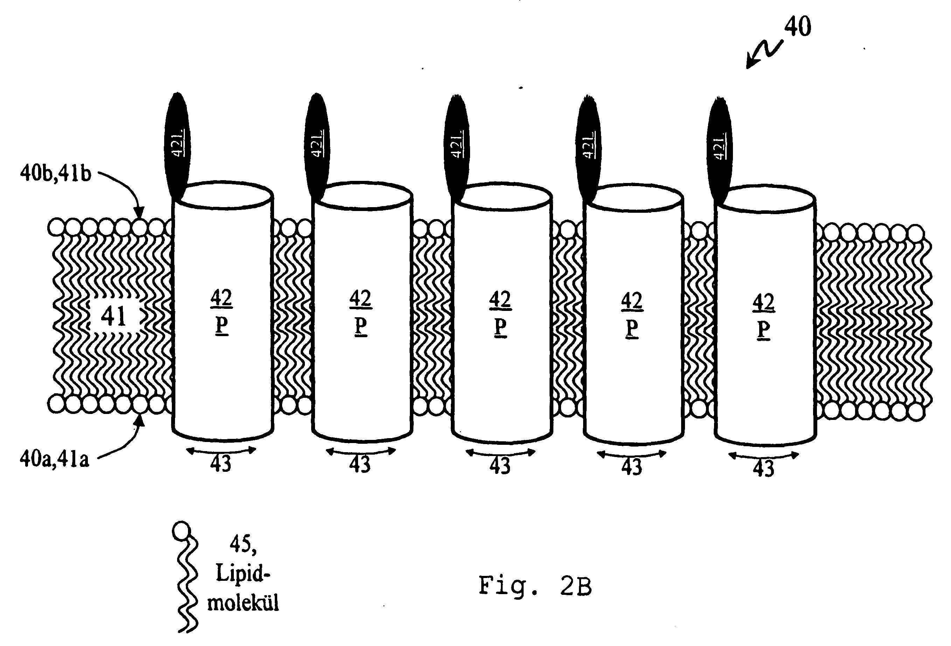

Das native oder durch Mutagenese bzw. chemische Derivatisierung modifizierte OmpG wird in künstliche Membranen eingebaut. Für schaltbare selektive Elektroden werden die rekonstituierten Membranen auf funktionalisierte Goldelektroden aufgetragen. Dazu wird die selektive, schaltbare Membran z.B. mit Hilfe von Thioalkanen an der Goldoberfläche gebunden (Fig. 1). Durch Variation des pH-Wertes der Messlösung werden die Poren des Wildtyp-Proteins entweder geschlossen oder geöffnet. Analog können Mutanten, die mit bestimmten Ionen oder anderen Substanzen schaltbar sind, in künstliche Membranen eingebaut und als selektive, schaltbare Sensoren und Elektroden verwendet werden. Für schaltbare Nanofilter werden die Membranen auf Milliporefilter aufgebracht (Fig. 2).

### Anwendungen von Wildtyp-OmpG

Wildtyp-OmpG kann ohne weitere Modifikation als Biosensor zur Detektion oder als Nanofilter für ungeladenen Moleküle, z.B. Zuckermoleküle bis zu einer Größe von ca. 1 kDa eingesetzt werden. Biosensor- oder Filterfunktion können durch Senkung des pH-Wertes oder Zugabe von Gd³⁺-Ionen abgeschaltet werden. Durch Anheben des pH-Wertes auf ca. 7,0 bzw. Zugabe von Chelatbildnern wie EDTA wird die Blockierung rückgängig gemacht.

### Veränderung der Kanaleigenschaften

Auf der Grundlage unserer neuen Röntgenstruktur können wir mit molekularbiologischen Standardmethoden auf einfache Weise OmpG-Mutanten mit gezielt zugeschnittenen Kanal- und Bindungseigenschaften entwickeln und herstellen. Da die Sequenz von OmpG keine Cysteine enthält, können durch Mutagenese reaktive SH-Gruppen an beliebiger Stelle in der Struktur eingeführt und anschließend chemisch derivatisiert werden, um so das Spektrum der Kanaleigenschaften und der erkannten Moleküle zu erweitern.

### Permanent offene Poren

Als einfachste Modifikation kann ein permanent offener Kanal ohne Schaltfunktion durch Entfernen der Schleife L6 bzw. durch Ersetzten der Histidine des pH-Sensors erhalten werden.

### OmpG-Mutanten mit Spezifizität für Metallionen

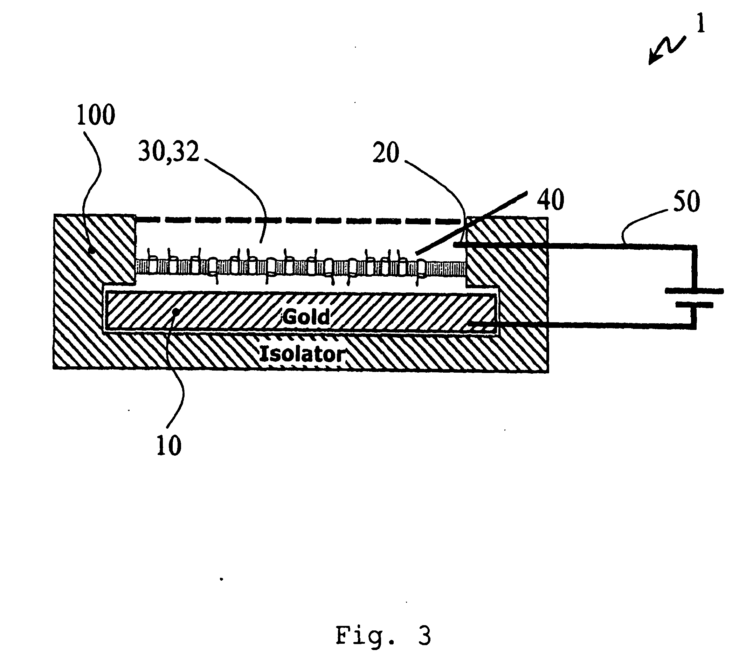

Als Modifikation werden Bindungsstellen für divalente und/oder trivalente Kationen in die Schleife L6 oder die ebenfalls flexiblen Schleifen L1, L2 und L7 und an geeigneten Stellen an der Innenwand des Zylinders eingebaut. Um eine Zn-Bindungsstelle einzuführen, wird zum Beispiel Glu227 in Schleife L6 durch Histidin ersetzt, das in der geschlossenen Form mit His231 (vom pH-Sensor) ein Histidinpaar bilden kann. Auf der Zylinder-Innenseite werden dann Phe269 und Glu17 oder Val19 durch Cysteine ersetzt. Dadurch entsteht eine Bindungsstelle für Zn²⁺ aus zwei Histidinen und zwei Cysteinen in geeigneter Konformation, so dass bei Gegenwart von geringer Zn²⁺- z.B. im Bereich von etwa 1 pmol/l bis etwa 1 µmol/l - der Kanal schließt (Fig. 3). Durch Zugabe von Chelatbildnern wie z.B. EDTA wird Zn²⁺entfernt und der Kanal öffnet sich.

Als Alternative werdenIle226 und Arg228 in L6 durch Histidine sowie Thr64 und Phe66 auf der gegenüberliegenden Zylinderwand durch Cysteine ersetzt. Auch dadurch würde eine Zn²⁺-Bindungsstelle entstehen. Analog wird für Bindungsstellen anderer toxischer Schwermetallionen. z.B. Cu⁺, Hg²⁺, Cd²⁺, Pb²⁺ verfahren.

Die so modifizierten Poren können als stochastische Sensoren eingesetzt werden, die Metallionen nicht nur detektieren sondern gleichzeitig auch quantifizieren. Im Prinzip kann die Art und Konzentration der Ionen durch Messungen der Fluktuationen des Stroms bestimmt werden, der durch den modifizierten OmpG-Kanal fließt.

### Verwendung im Wirkstoff-Screening

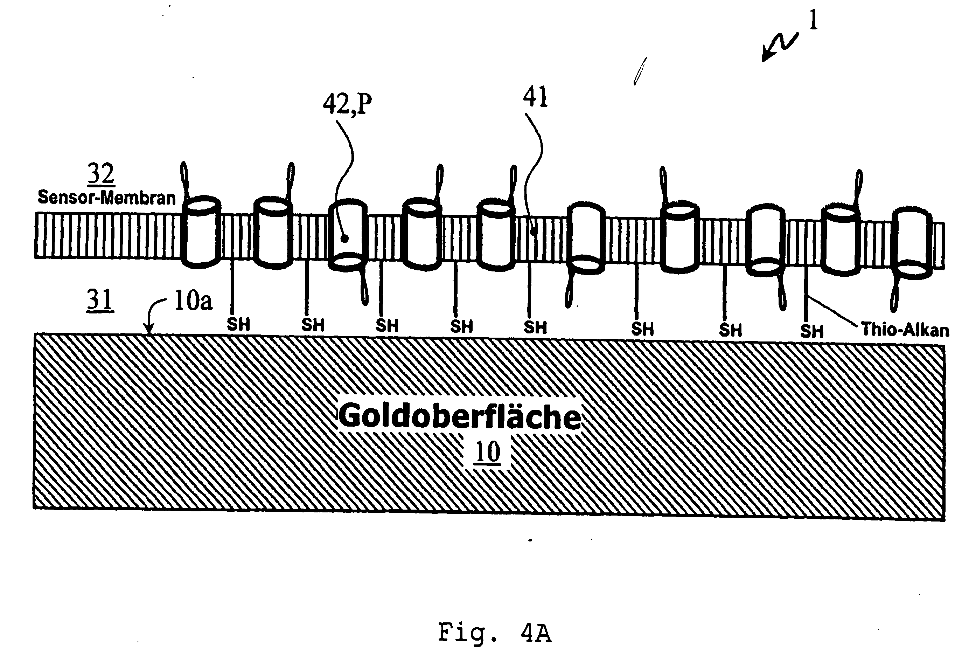

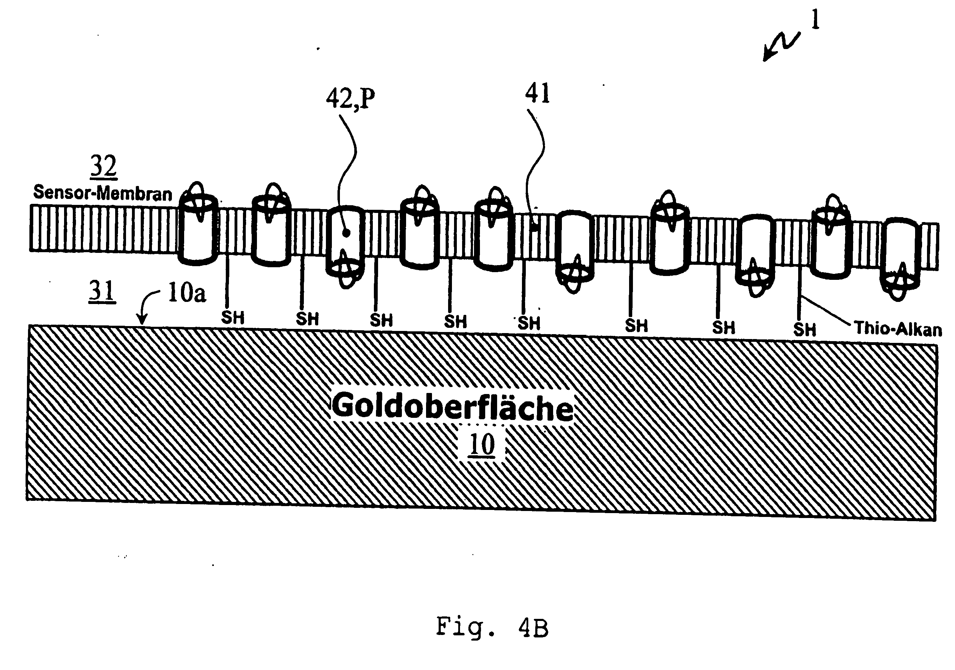

In die DNA-Sequenz, welche die Porenschleife L6 codiert, oder in andere geeignete Teile der OmpG-Gensequenz werden Restriktions-Kassetten eingebaut, mit denen durch Zufallsmutagenese eine große Anzahl beliebiger Polypeptidsequenzen eingefügt werden (Fig. 4). OmpG-Moleküle mit diesen Sequenzen werden rückgefaltet und mit Wirkstoff-Bibliotheken auf Bindung bzw. Transport von pharmakologisch oder medizinisch relevanten Substanzen getestet. Hierfür wird ein Hochdurchsatz-Verfahren entwickelt, mit dem die Arbeitsschritte und die Auswertung in 96er-Platten durchgeführt werden können. Ziel ist es dabei, OmpG-Mutanten zu identifizieren, die als schaltbare Kanäle oder Sensoren für bestimmte Substanzen in biomedizinischen Anwendungen nutzbar gemacht werden können.

### Einsatz in der zellbiologischen Forschungz

Es können erzeugte OmpG-Mutanten mit bestimmten Schaltfunktionen werden in transformierten Zellen bzw. Organismen exprimiert. Es werden OmpG-Mutanten gewählt, die in Anwesenheit einer ubiquitär vorhandenen Schaltsubstanz (z.B. Ca²⁺) geschlossen und daher in diesem Zustand für die Zelle unschädlich sind. Dadurch sollte sich, wie in vergleichbaren Fällen (z.B. bei intrazellulärer Expression von grün fluoreszierendem Protein. GFP) vermeiden lassen, dass das exprimierte Protein in der Zelle abgebaut wird. Hilfreich dürfte hierbei die Tatsache sein, dass es sich bei OmpG um ein ungewöhnlich stabiles und robustes Membranprotein handelt. Außerdem werden für den beabsichtigten Effekt nur wenige in die Membran eingebaute OmpG-Moleküle benötigt. Durch Entfernen der Schaltsubstanz (z.B. durch Zugabe von EDTA) wird die Pore geöffnet, was zunächst zu einer schaltbaren, prinzipiell reversiblen Depolarisation der Zellmembran führt. Wenn die Pore geöffnet bleibt, führt das zum Tod der Zellen, die das modifizierte OmpG exprimieren.

### Einsatz in der Krebstherapie

OmpG-Mutanten einer Form, die permanent offen ist werden als Fusionsproteine mit Antikörpern, die bestimmte Tumorzellen erkennen, injiziert und am Zielort in die Zellmembran eingebaut. Die Poren werden in die Zielmembran eingebaut und die Tumorzellen sterben ab. Alternativ können zugängliche Tumorzellen mit geeigneten Virenvektoren so transformiert werden, dass sie das permanent offene OmpG exprimieren, so dass sie selektiv abgetötet werden.

### Mutation der Histidine des pH-Sensors

Es kommen in erster Linie Aminosäuren mit geladenen Seitenketten (Aspartat. Glutamat, Arginin. Lysin) oder Cystein in Frage. Für biotechnologische Zwecke unter

Umständen auch interessant sind Aromaten (Phenylalanin. Tryptophan, Tyrosin), hydrophobe Seitenketten (Methionin. Leucin. Isoleucin. Valin. Alanin), sowie Serin. Threonin. Asparagin. Glutamin, Prolin, Glycin.

Auch eine Einzelmutante H261D/E ist denkbar, weil diese auch eine H-Brücke zu H231 ausbilden könnte.

### Ersatz von His231 oder His261 durch Aspartat oder Glutamat

In der Mutante H231D bzw. H231E ist die Schleife L6 in der offenen Form stabilisiert. In der Mutante H261D bzw. H261E wäre dagegen bei neutralem pH die geschlossene Form bevorzugt, wegen Abstoßung der benachbarten negativen Ladung von Asp267. Eine Doppelmutante von His231 und His261 zu Aspartat (D) oder Glutamat (E) würde bei neutralem pH wegen der negativen Ladungen der Carboxyreste die Pore schließen, und bei saurem pH unter 4 die Pore öffnen.

### Ersatz von His231 oder His261 durch ein Ionenpaar

Wenn Histidin 231 durch eine saure Gruppe (Aspartat oder Glutamat) und Histidin 261 Durch eine basische Gruppe (Arginin oder Lysin) ersetzt wird (oder umgekehrt), können sich Salzbrücken ausbilden, welche die Pore in Abhängigkeit von der Ionenstärke des Mediums öffnen oder schließen.

### Ersatz von His231 oder His261 durch Cystein

Durch Mutation von beiden Histidinen zu Cystein würde ein Redox-Sensor geschaffen, wobei die Pore unter oxidierenden Bedingungen durch Ausbildung einer kovalenten S-S-Brücke offen, unter reduzierenden Bedingungen, besonders in Gegenwart freier SH-Gruppen jedoch potentiell geschlossen ist.

Alternativ kann ein Redoxsensor so gebaut werden, dass Glu227 in Schleife L6 und Phe269, Glu17 oder Va119 auf der Innenseite der Pore durch Cystein ersetzt werden. Durch Ausbildung einer kovalenten S-S-Brücke zwischen Schleife L6 und der Zylinderwand würde die geschlossene Form unter oxidierenden Bedingungen fixiert, und unter reduzierenden Bedingungen geöffnet.

### Konzentrationsbereich von Metallionen für den Biosensor

In Escherichia coli beträgt die Gesamtmenge von Ca. Zn und Fe ca. 2·10⁵ Atome pro Zelle, entsprechend etwa 0.1 mmol/1. Kupfer und Mangan haben eine Konzentration von 10 µmol/l bis 100 µmol/l. Kobalt, Nickel. Vanadium kommen in Spuren (einige µmol/l) vor. Die Konzentration der freien Ionen liegt jeweils um ein Vielfaches darunter. Ionenbindungsstellen in Proteinen müssen eine Affinität aufweisen, die bei diesen geringen Konzentrationen noch eine nahezu vollständige Besetzung mit dem jeweiligen Ion gewährleistet. Es wird daher davon ausgegangen, dass eine Bindungsstelle, die wir in Anlehnung an natürlich vorkommende Ionenbindungsstellen in OmpG einbauen, eine ähnliche Affinität aufweisen wird, und auf Ionenkonzentrationen im Bereich von 1 pmol/l bis 1 µmol/l reagiert.

### Biomedizinische Einsatzmöglichkeiten von OmpG

U.a. die folgenden Einsatzmöglichkeiten auf dem Gebiet der Biomedizin ergeben sich aus der Eigenschaft der Porine, sich spontan in Zielmembranen zu integrieren:

### Kontrollierte Permeabilitätsänderung von Membranen

Die Integration von modifiziertem Außenmembranporin G in die Zellmembran ermöglichst es im Prinzip, diese gezielt permeabel zu machen. In die Zellmembran integriertes Außenmembranporin G ermöglicht den Eintritt von Gefrierschutzmitteln in Säugerzellen, wodurch diese für die Lagerung bei tiefen Temperaturen leichter eingefroren und weniger geschädigt werden.

### Wirkstofftransport

Die Effizienz vieler potentiell hoch wirksamer Medikamente ist durch deren Transport zum Zielort sowie eine schwer kontrollierbare Dosierung begrenzt. Beide Probleme können prinzipiell dadurch gelöst werden, dass Medikamente, Enzyme und andere therapeutisch wirksame Substanzen in Liposomen zu verpacken, in die schaltbares rekombinantes Außenmembranporin G integriert wurde, sowie Moleküle, welche die Zielzellen erkennen. Mit Auslösefaktoren, welche die Poren öffnen, wäre dann eine Ausschüttung der Wirkstoffe und somit eine geregelte Dosierung am Zielort möglich.

### Krebstherapie

Ungefaltetes OmpG wird an spezifische Antikörper gekoppelt, die Tumorzellen anhand ihrer Antigene erkennen und so das Porin in Kontakt mit der Oberfläche der Zielzellen bringen. Das Porin integriert sich in die Zielmembran, wodurch die Zelle entweder direkt zerstört oder anfällig für Chemotherapeutika wird. Ein ähnlicher Ansatz wurde mit rekombinanten Toxinen (bisher jedoch nicht mit Porinen) in klinischen Tests der Phase I und II erprobt. Als Beispiel sei die Behandlung von hemapoeitischen Tumoren mit Diphterietoxin und Pseudomonasexotoxin genannt. Die Toxine waren mit Interleukin 2 fusioniert, welches T und B Zellen erkennt und diese in die Lage versetzt. Krebszellen anzugreifen: [1], [2].

### Verwendung von Mutanten

Bei der Struktur der erfindungsgemäßen Anordnung für eine funktionelle Membran sowie bei sämtlichen Anwendungen und Verwendungen der erfindungsgemäßen Anordnung für eine funktionelle Membran, insbesondere im Bereich Sensoranordnungen, Filteranordnungen, Permeabilitätssteuerung und Krebstherapie, können neben Wildtypporineinheiten auch Mutanten vorgesehen sein, insbesondere die Mutanten und beliebig kombinierten oder gemischten Mehrfachmutanten H231D, H231E, H231C, H231K. H231R, H261D. H261E, H261C, H261K, H261R und deren Derivate in Frage, insbesondere diejenigen des Außenmembranporins G aus dem Bakterium Escherichia coli.

Nachfolgend wird die vorliegende Erfindung anhand einer schematischen Zeichnung auf der Grundlage bevorzugter Ausführungsbeispiele näher erläutert.
- **Fig. 1A. 1B**: zeigen in schematischer und geschnittener Seitenansicht As- pekte einer erfindungsgemäß vorgesehenen funktionellen Membran mit stochastisch ausgerichtet vorgesehenen OmpG- Molekülen in geöffnetem bzw. in geschlossenem Zustand.
- **Fig. 2A, 2B**: zeigen in schematischer und geschnittener Seitenansicht As- pekte einer erfindungsgemäß vorgesehenen funktionellen Membran mit orientiert vorgesehenen OmpG-Molekülen in ge- öffnetem bzw. in geschlossenem Zustand.
- **Fig. 3**: ist eine schematische und geschnittene Seitenansicht, welche eine erste Ausführungsform einer erfindungsgemäßen Sensor- anordnung zeigt.
- **Fig. 4A**: ist eine schematische und geschnittene Seitenansicht, welche Details der Ausführungsform aus Fig. 1 zeigt, wobei OmpG im geöffneten Zustand vorliegt.
- **Fig. 4B**: ist eine aschematische und geschnittene Seitenansicht, welche Details der Ausführungsform aus Fig. 1 zeigt, wobei OmpG im geschlossenen Zustand vorliegt.
- **Fig. 5**: zeigt in schematischer und perspektivischer Seitenansicht eine erste Ausführungsform der erfindungsgemäßen Filteranord- nung.
- **Fig**. **6**: ist eine schematische und geschnittene Seitenansicht, welche Details der Ausführungsform aus Fig. 5 erläutert.
- **Fig. 7A, 7H**: ist eine schematische Draufsicht auf ein OmpG-Molekül mit Zn²⁺-speziftschem Verhalten in geschlossenem bzw. in geöffne- tem Zustand.
- **Fig. 8A, 8B**: sind schematische und perspektivische Seitenansichten auf Ausführungsformen der funktionellen Membran mit OmpG- Molekül mit Spezifizität in Bezug auf eine gegebene Substanz Z in geöffnetem bzw. in geschlossenem Zustand.
- **Fig. 9a- 13d**: zeigen in schematischer Form jeweils Seitenansichten oder Draufsichten molekularer Modelle des OmpG-Moleküls in ge- öffnetem bzw. in geschlossenem Zustand.
- **Fig. 14**: zeigt in schematischer Form die Anordnung des OmpG- Moleküls auf atomarer Ebene in seiner nativen Umgebung.
- **Fig. 15A - C**: zeigen in schematischer Form die kristalline Anordnung von OmpG-Molekülen, wie sie zur Durchführung der Röntgenstruk- turanalyse verwendet werden.

Nachfolgend werden gleiche oder strukturell und/oder funktionell ähnliche Elemente und Komponenten mit denselben Bezugszeichen bezeichnet, ohne dass in jedem Fall ihres Auftretens eine detaillierte Beschreibung wiederholt wird.

Vor der detaillierten Erläuterung der Anwendungsaspekte der vorliegenden Erfindung, werden anhand der Fig. 1A, 1B, 2A und 2B grundlegende strukturelle Aspekte der vorliegenden Erfindung im Detail erläutert.

Sämtliche Fig. 1A bis 2B zeigen in schematischer und geschnittener Seitenansicht eine funktionelle Membran 40, wie sie erfindungsgemäß Verwendung findet.

Bei sämtlichen Ausführungsformen besteht die funktionelle Membran 40 zum einen aus einer Lipiddoppelschichtmembran 41. durch welche eine Vorderseite 40a, 41a sowie eine Rückseite 40b, 41 b für die funktionelle Membran 40 bzw. für die Lipiddoppelschichtmembran 41 definiert wird. Diese Lipiddoppelschichtmembran wird durch Lipidmoleküle 45 gebildet, die sich in einem wässrigen Medium aufgrund ihrer amphiphilen Eigenschaften spontan in einer Doppelschichtstruktur organisieren und somit jeweils eine zweidimensionale Flüssigkeit bilden. Eingebettet in diese Lipiddoppelschichtmembran 41 sind Proteinmoleküle 42, welche die Lipiddoppelschichtmembran 41 membrandurchspannend und funktionell durchdringen.

Erfindungsgemäß werden die Membranproteine 42 gebildet von dem Außenmembranporin G aus dem Bakterium Escherichia Coli oder aus Derivaten davon. Dieses Membranprotein 42 bildet grundsätzlich eine Pore 43. durch welche die Vorderseite 40a, 41a und die Rückseite 40b. 41b miteinander verbunden werden können. Die Pore 43 de Außenmembranporins G kann durch einen bestimmten Mechanismus, nämlich einen zu Konformationsänderung fähigen Loop 42L in der Sequenz des Außenmembranporins G 42 in einen geöffneten und in einem geschlossenen Zusatz versetzt werden, und dies, wie oben bereits im Detail dargelegt wurde, in gesteuerter Art und Weise, nämlich in Abhängigkeit vom pH-Wert und/oder von bestimmten Ionenkonzentrationen im wässrigen Medium.

In der in den Fig. 1A und 1B gezeigten Anordnung für die erfindungsgemäße funktionelle Membran 40 sind die Moleküle 42 des Außenmembranporins G hinsichtlich ihrer Orientierung statistisch verteilt in der Lipiddoppelschichtmembran 41 eingebaut. Das bedeutet, dass mit einer bestimmten Verteilung die Orientierungen der einzelnen Moleküle 42 in der Lipiddoppelschichtmembran 41 vorliegen. In der in den Fig. 1A und 1B gezeigten Situation sind von links nach rechts die Moleküle 1, 2 und 3 mit dem als Porenverschlussmechanismus dienenden Loop 42L in Richtung auf die Rückseite 40b, 41 b der funktionellen Membran 40 und der Lipiddoppelschichtmembran 41 hin orientiert, wogegen die dritten und fünften Moleküle mit diesem als Verschlussmechanismus dienenden Loop 42L zur Vorderseite 40a. 41a der funktionellen Membran bzw. der Lipiddoppelschichtmembran 41a zeigen.

In der Anordnung der Fig. 1A sind sämtliche Poren 43 der Moleküle 42 des Außenmembranporins G durch Eingeklapptsein oder durch Einstülpen des Loops 42L verschlossen. Bei der Fig. 1B dagegen sind durch eine entsprechende Wahl der Bedingungen im wässrigen Medium sämtliche Poren 43 der Moleküle 42 des Außenmembranporins durch eine entsprechende Konformationsänderung im als Verschlussmechanismus dienenden Loop 42L geöffnet.

Die Fig. 2A und 2B stellen eine mit den Fig. 1A bzw. 1B beschriebenen Situation analoge Anordnung dar, wobei jedoch durch bestimmte Mechanismen ein Großteil oder sämtliche Moleküle 42 des Außenmembranporins G in der funktionellen Membran 40 und somit in der Lipiddoppelschichtmembran 41 derart orientiert eingebaut sind, dass sämtliche Moleküle 42 mit dem als Verschlussmechanismus dienenden Loop 42L zur Rückseite 40b, 41 b der funktionellen Membran 40 bzw. der Lipiddoppelschichtmembran 41 hin zeigen. Auch hier sind in der Fig. 2A sämtliche Poren 43 verschlossen, wogegen in der Anordnung der Fig. 2B sämtliche Poren 43 durch eine entsprechende Konformationsänderung des jeweils als Verschlussmechanismus dienenden Loops 42L geöffnet sind.

Selbstverständlich ist es auch denkbar, dass sämtliche Moleküle 42 des Außenmembranporins G mit dem als Verschlussmechanismus dienenden Loop 42L zur Vorderseite 40a, 41a der funktionellen Membran 40 bzw. der Lipiddoppelschichtmembran 41 hin orientiert sind und werden.

Die Fig. 3 zeigt in schematischer und geschnittener Seitenansicht eine Sensoranordnung 1, und zwar in einer Anwendung als Biosensor.

Dabei dient die funktionelle Membran 40 als so genannte Sensormembran. Die gesamte Sensoranordnung 1 ist in oder mit einem Gehäuse 100 ausgebildet, in dessen Inneren ein Hohlraum vorgesehen ist. Das Gehäuse besteht aus einem Isolator. Der Hohlraum ist auf einer seiner Unterseiten mit einer ersten Elektrodeneinrichtung 10 aus Gold versehen, die gleichzeitig als Bodenbereich dient. Über der Oberfläche 10a der ersten Elektrodeneinrichtung erstreckt sich der eigentliche Messraum. Dieser ist mit einem wässrigem Messmedium 30 gefüllt. Direkt benachbart zur Oberfläche 10a der ersten Elektrodeneinrichtung 10 ist die funktionelle Membran 40 als Sensormembran ausgebildet. Diese funktionelle Membran 40 besitzt eine der in den Fig. 1A bis 2B gezeigten Strukturen. Das bedeutet, dass die funktionelle Membran 40 aus der Anordnung der Fig. 3 auf der Grundlage einer Lipiddoppelschichtmembran 40 aus Lipidmolekülen 45 besteht, die membrandurchspannend. Membranproteine 42 in Form von Außenmembranporinen G in funktioneller Form aufweist. In der in Fig. 3 gezeigten Darstellung wird durch die funktionelle Membran 40 das wässrige Messmedium 30 im Innenraum des isolierenden Gehäuses 100 in zwei Kompartimente 31 und 32 unterteilt. Das direkt an der Oberfläche 10a der ersten Elektrodeneinrichtung 10 befindliche erste Kompartiment 31 des wässrigen Messmediums 30 liegt somit auf der Vorderseite 40a der funktionellen Membran 40. wogegen das von der Oberfläche 10a der ersten Elektrodeneinrichtung 10 abgewandte zweite Kompartiment 32 auf der Rückseite 40b der funktionellen Membran liegt.

Die funktionelle Membran 40 kann in der Anordnung der Fig. 3, obwohl dies hier im Detail nicht dargestellt ist, auf der Oberfläche 10a der ersten Elektrodeneinrichtung 10 durch eine Verankerung immobilisiert sein.

In der in Fig. 3 gezeigten Darstellung sind sämtliche Membranproteine 42 in Form der Außenmembranporine G stochastisch orientiert in der funktionellen Membran 40 angeordnet und befindet sich hier im geöffneten Zustand. Der Öffnungszustand kann erfindungsgemäß in gesteuerter Art und Weise eingestellt werden. Auch ist eine Orientierung oder eine andere als die in Fig. 3 dargestellte stochastische Verteilung in Bezug auf die Orientierung der Transmembranproteine 42 in Form Außenmembranporine G denkbar.

Die als Goldelektrode fungierende erste Elektrodeneinrichtung 10 ist an einen äußeren Messkreis 50 angeschlossen. Dieser kann eine Stromquelle, eine Spannungsquelle und/oder entsprechende Nachweisinstrumente. z.B. zeitlich hochauflösende Strom-/Spannungsmesseinrichtungen aufweisen. Zum Schließen des Messkreises 50 ist eine zweite Elektrodeneinrichtung 20 vorgesehen, die als Gegenelektrode fungiert und in der in Fig. 3 gezeigten Anordnung mit dem zweiten Kompartiment 32 des wässrigen Messmediums 30 in elektrischem Kontakt steht. Denkbar sind hier Silber-Silberchloridelektroden oder auch Platinelektroden.

Die Fig. 4A und 4B zeigen in größerem Detail noch einmal die Anordnung der erfindungsgemäßen Sensoranordnung aus Fig. 3. Hier sind ausschließlich das Messmedium 30 mit den ersten und zweiten Kompartimenten 31 bzw. 32, die erste Elektrodeneinrichtung 10 in Form einer Goldelektrode sowie die an der Oberfläche 10a der ersten Elektrodeneinrichtung 10 immobilisierte funktionelle Membran 40 dargestellt.

In der Fig. 4A sind sämtliche Transmembranproteine 42 in Form der Außenmembranporine G im geöffneten Zustand dargestellt, jeweils mit stochastischer Verteilung in Bezug auf ihre Orientierung in der zugrunde liegenden Lipiddoppelschichtmembran 41. In der Fig. 4B sind dagegen sämtliche Transmembranproteine in der Form Außenmembranporine G in ihrem geschlossenen Zustand dargestellt. Das bedeutet, in der Fig. 4A sind sämtliche Poren 43 geöffnet, in der Fig. 4B dagegen sind sie geschlossen.

Die Immobilisierung der funktionellen Membran 40 und insbesondere der Lipiddoppelschichtmembran 41 auf der Oberfläche 10a der ersten Elektrodeneinrichtung 10 erfolgt durch eine spezifische Wechselwirkung der so genannten Thiogruppe oder SH-Gruppe mit dem Metallgold. Dazu kann die Lipiddoppelschichtmembran 41 mit so genannten Thioalkanen oder Thiolipiden versehen werden, d.h. also letztlich mit Elementen, die z.B. eine lange aliphatische Kette mit einer endständigen SH-Gruppe als funktioneller Gruppe aufweisen. Es bildet sich durch spezifische Wechselwirkung dabei eine Art kovalente Bindung zwischen der SH-Gruppe und den Oberflächenatomen der Oberfläche 10a der Goldelektrode 10 aus.

Dabei kann die Lipiddoppelschichtmembran 41 durch Hinzufügen der Thioalkane oder der Thiolipide asymmetrisch oder symmetrisch aufgebaut sein, so dass entweder nur die Vorderseite 40a der funktionellen Membran 40 die SH-Gruppen aufweist, oder beide Seiten 40a und 40b.

Denkbar ist auch die Verwendung anderer funktioneller Gruppen und/oder die Verwendung anderer edler oder unedler Metalle. Denkbar ist insbesondere die Verwendung von Silberelektroden oder Platinelektroden als erste Elektrodeneinrichtung 10.

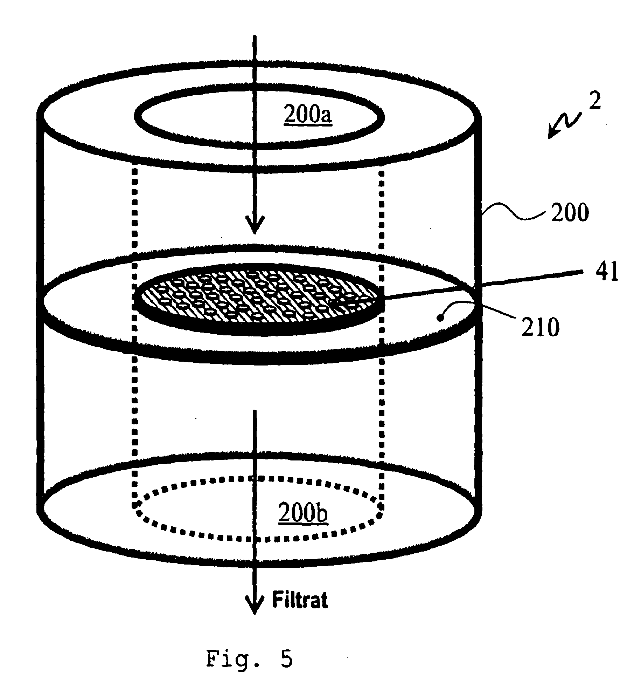

Die Fig. 5 zeigt eine Ausführungsform der erfindungsgemäßen Filteranordnung 2. Diese besteht aus einem hohlzylindrischen Filtergehäuse 200. Es ergibt sich somit eine Art Rohr, durch welches in Pfeilrichtung ein Flüssigkeitsstrom hindurchgetrieben werden kann. In der Mitte befindet sich ein Septum 210, also eine Trennwand, welche das als Rohr dienende Gehäuse 200 und dessen Inneres in einen stromaufwärts gelegenen Teil 200a und in einen stromabwärts gelegenen Teil 200b unterteilt. Das Septum 210 beherbergt eine funktionelle Membran oder eine Mehrzahl funktioneller Membranen 40 im Sinne der Erfindung. Die jeweils vorgesehenen funktionellen Membranen 40 dienen somit als Filtermembranen. Durch die Spezifizität hinsichtlich der Permeabilitätseigenschaften, wie sie oben beschrieben wurden, werden beim Durchströmen des Inneren des Filtergehäuses 200 in Pfeilrichtung gemäß Fig. 5 bestimmte im Messmedium 30 enthaltene Bestandteile durch die funktionellen Membranen 40 hindurchgelassen, während andere zurückgehalten werden.

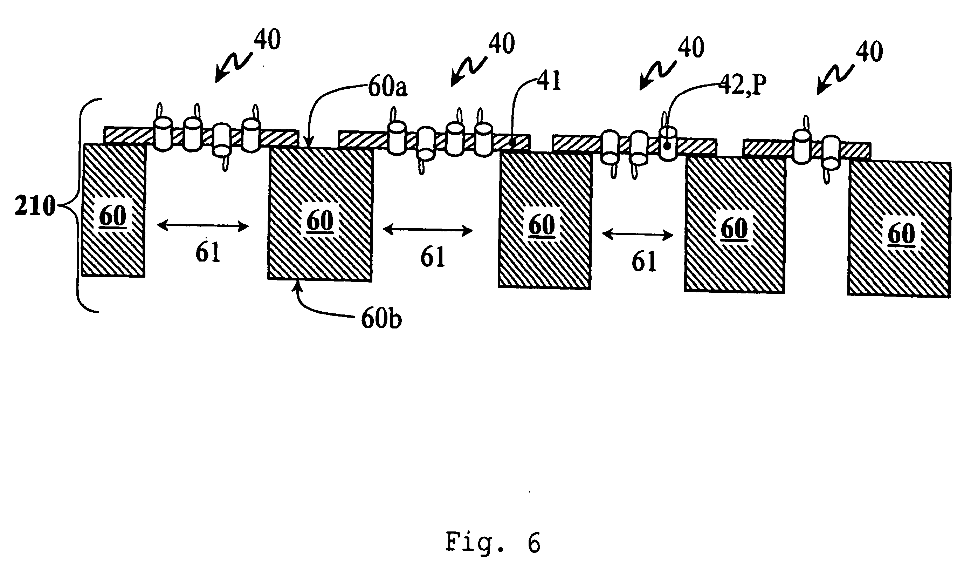

Die Fig. 6 zeigt in schematischer und geschnittener Seitenansicht Details der Filteranordnung 2 aus Fig. 5. Grundlegend ist dabei das Vorsehen eines Stützkörpers 60 als Septum 210 oder als Teil davon. Dieser Stützkörper 60 besitzt eine Oberseite 60a, die stromaufwärts gelegen ist und somit vom anströmenden Messmedium 30 im Betrieb der erfindungsgemäßen Filteranordnung 2 angeströmt wird. Dieser Oberseite 60a gegenüberliegend ist eine Rückseite 60b vorgesehen. Des Weiteren weist der Stützkörper 61 auf, die voneinander lateral beabstandet im Stützkörper 60 ausgebildet sind und die Vorderseite 60a mit der Rückseite 60b verbinden. Jede Pore wird hinsichtlich ihrer Querschnittsfläche durch eine funktionelle Membran 40 oder durch einen Teil einer funktionellen Membran abgedeckt, so dass die Poren 61 ausschließlich durch die viel feiner strukturierten steuerbaren Poren 43 der Außenmembranporine G als Transmembranproteine 42 der funktionellen Membran 40 in ihrer Durchlässigkeit oder Permeabilität gesteuert werden. Dies ist in Fig. 6 dargestellt.

Als Resultat ergibt sich, dass man auf der Grundlage eines als herkömmlichen Filter dienenden Stützkörpers 60 durch Zusatz der funktionellen Membran 40 eine Nanofilteranordnung schaffen kann, die eine viel stärker reduzierte Ausschlussgröße oder ein viel geringeres Ausschlussvolumen hinsichtlich der Filtereigenschaften besitzt.

In der Anordnung der Fig. 6 ist jede Pore 61 des Stützkörpers 60 durch genau eine funktionelle Membran 40 im Sinne der Erfindung abgedeckt. Denkbar ist aber auch, das dies durch mehrere funktionelle Membranen 40 geschieht, z.B. in Form eines Stapels. Die als Transmembranproteine 42 vorgesehenen Außenmembranporine G sind hinsichtlich ihrer Orientierung in der Lipiddoppelschichtmembran 41 stochastisch angeordnet und in der Ausführungsform der Fig. 6 in ihrem geöffneten Zustand dargestellt. Jedoch können sämtliche in den Fig. 1A bis 2B gezeigten und damit im Zusammenhang beschriebenen Anordnungen in der Anordnung der Fig. 6 ausgetauscht werden.

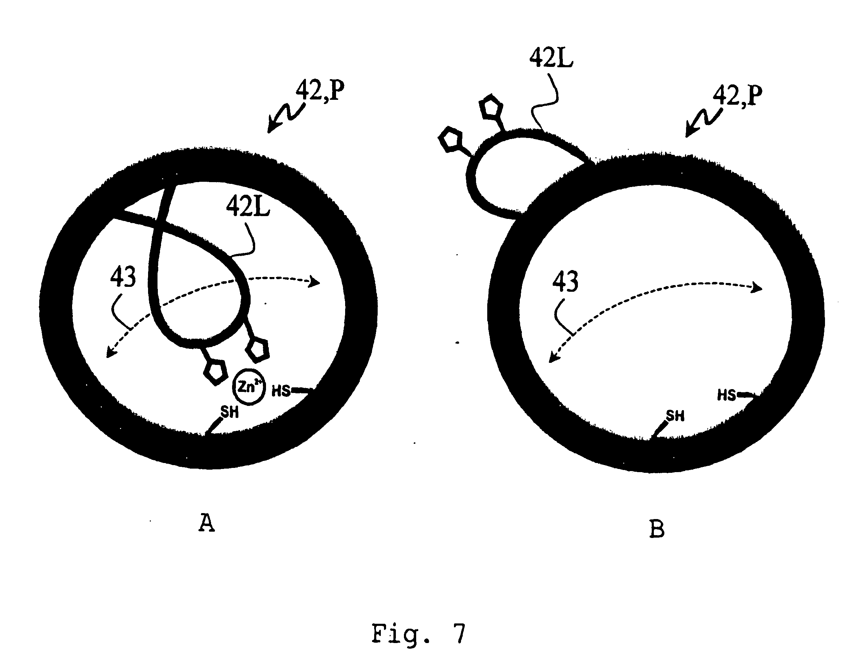

Die Fig. 7A und 7B zeigt in schematischer Draufsicht das als Transmembranprotein 42 fungierende Außenmembranporin G aus Escherichia coli im geschlossenen bzw. im geöffneten Zustand. Dabei ist der als Verschlussmechanismus dienende Loop 42L in der Sequenz zu Außenmembranporin G einmal in die Pore 43 hinein geklappt bzw. aus der Pore 43 hinaus geklappt, um diese entweder zu verschließen bzw. freizugeben. Das Verschließen bzw. Öffnen der Pore 43 des Transmembranproteins 42 erfolgt also über eine Konformationsänderung des Loops 42L. Dieser wird in der Ausführungsform der Fig. 7 gesteuert durch die Anwesenheit bzw. Abwesenheit von Zinkkationen. Dabei wird durch eine Zinkbindung, nämlich eine Bindung von Zn²⁺-Ionen die Konformationsänderung erzwungen bzw. durch Abpufferung oder Chelatbildung der Zn²⁺-Ionen, die dann nicht zur Bindung in der Pore 43 zur Verfügung stehen, aufgehoben. Dies ist auch analog für andere Metallionen modellierbar.

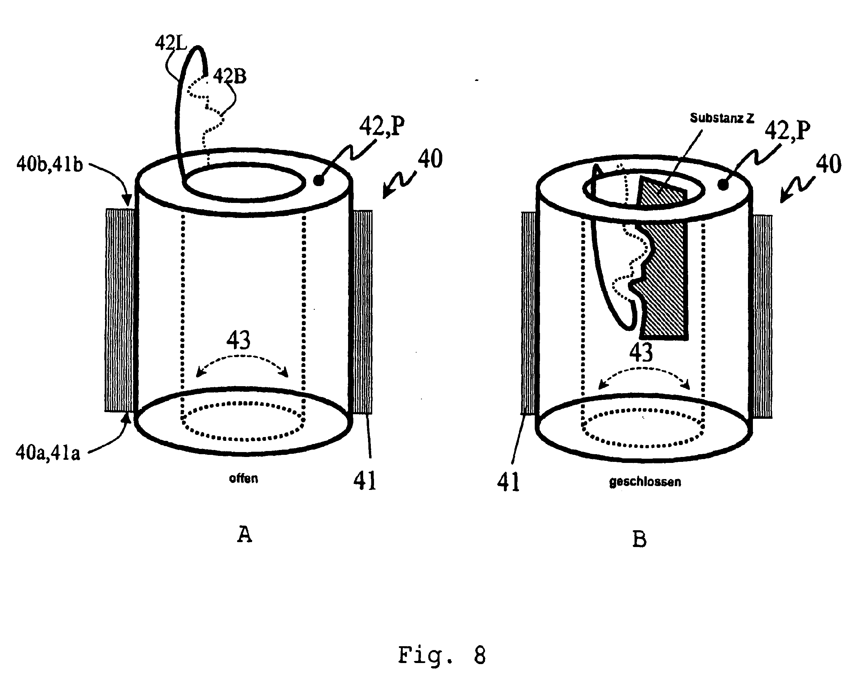

In der Ausführungsform der Fig. 7 wird die Zinkbindungsstelle definiert durch zwei Histidinreste in der Schleife L6 als Verschlussmechanismus dienender Loop 42L einerseits und durch zwei Cysteinreste - gekennzeichnet durch die SH-Gruppen im inneren der Pore 43 - so dass sich in Anwesenheit von Zinkionen die Konformation der Fig. 7A und in deren Abwesenheit die geöffnete Version gemäß Fig. 7B einstellt. Die Fig. 8A und 8B zeigen, dass bei einer Ausführungsform der erfindungsgemäßen Filteranordnung 2 durch zielgerichtete Mutagenese der als Verschlussmechanismus dienende Loop 42L, z.B. aus der Schleife L6 aus Außenmembranporin G derart abgeändert werden kann, dass dort eine Bindungsstelle 42B definiert wird, gegebenenfalls in Kooperation mit bestimmten Strukturen im Inneren der Pore 43, so dass bei Anwesenheit einer Triggersubstanz Z durch Konformationsänderung die Pore 43 verschlossen wird, so wie das in Fig. 8B dargestellt ist, während in Abwesenheit der Triggersubstanz Z die ftückkonformation vorliegt, bei welcher der als Verschlussmechanismus dienende Loop 42L sich außerhalb der Pore 43 befindet und diese freigibt.

Durch zufallsgesteuerte Mutationen oder auch durch gezielte Experimente können entsprechende Wirkstoffbibliotheken erzeugt werden, um daraus Derivate des Außenmembranporins G als Transmembranproteine 42 für die erfindungsgemäße funktionelle Membran 40 auszubilden, die dann ganz spezifischen Anforderungen im Hinblick auf die An/Abwesenheit bestimmter Substanzen erfüllt.

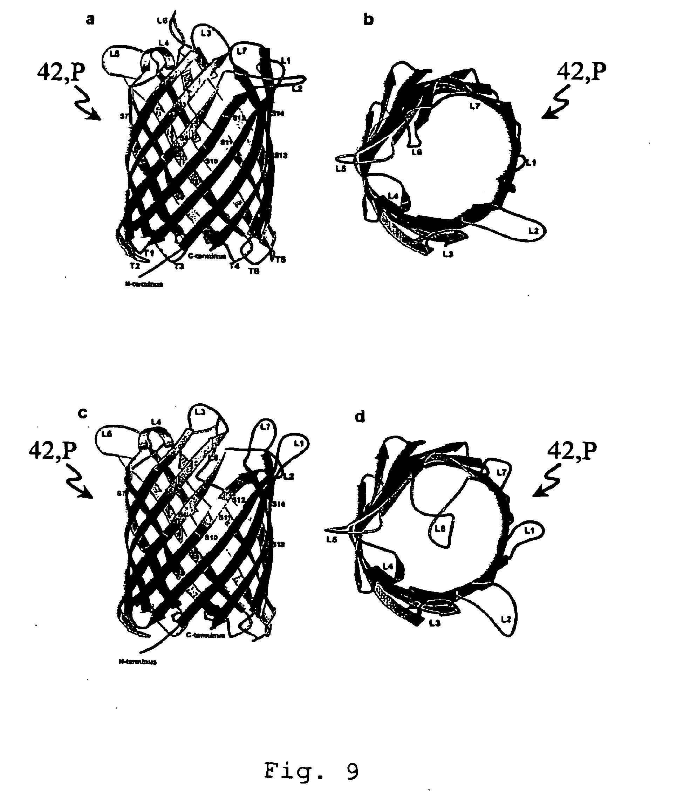

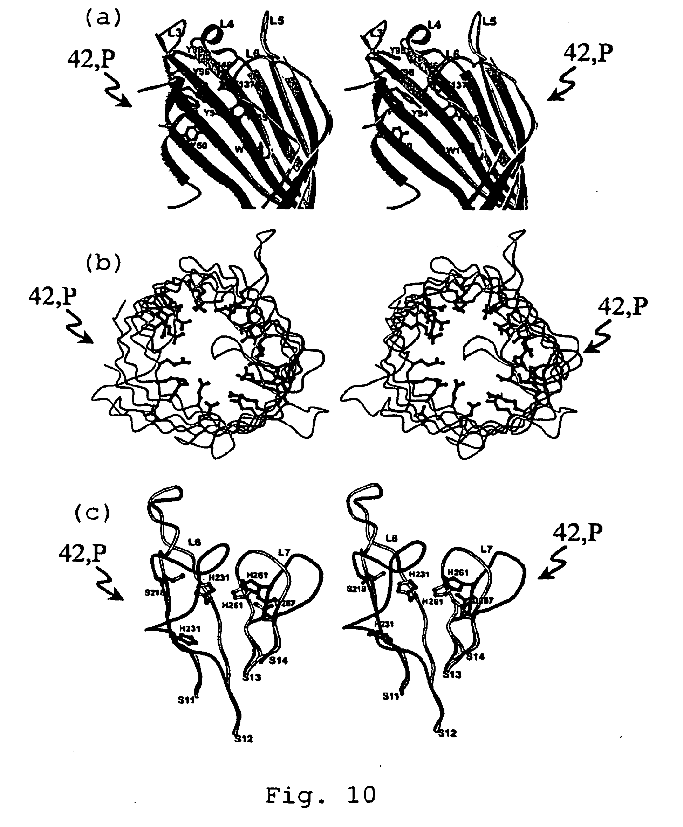

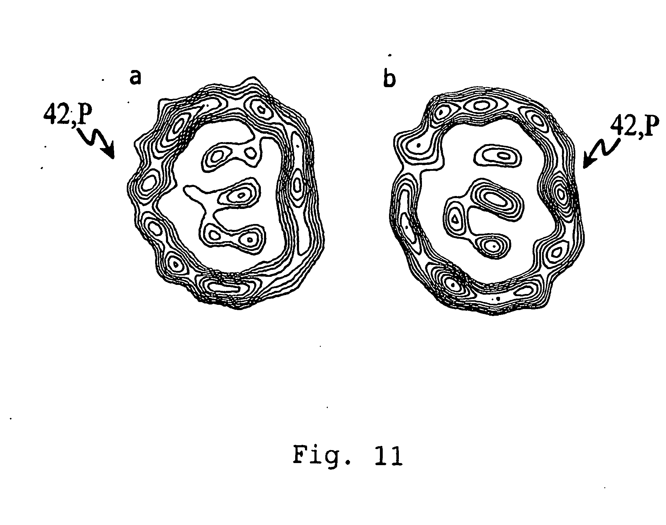

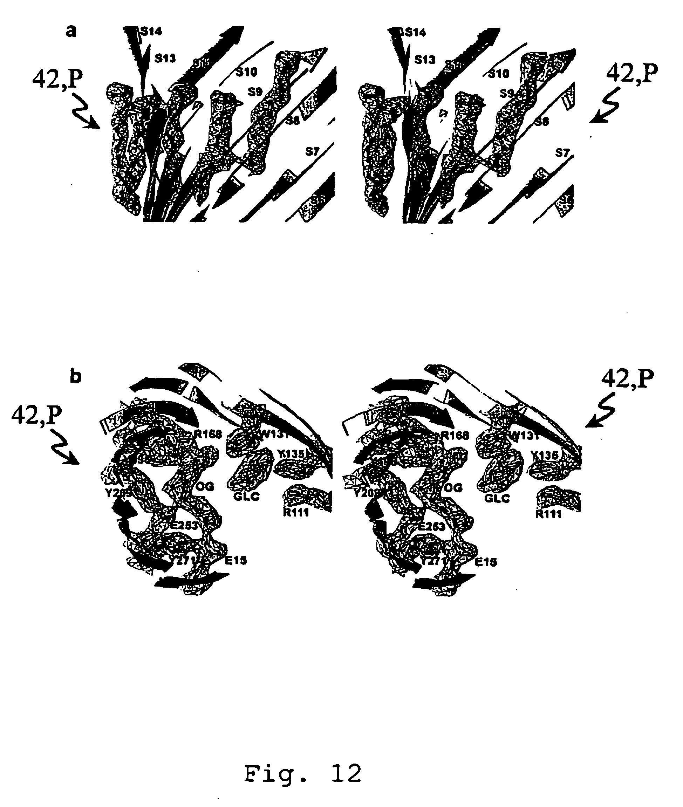

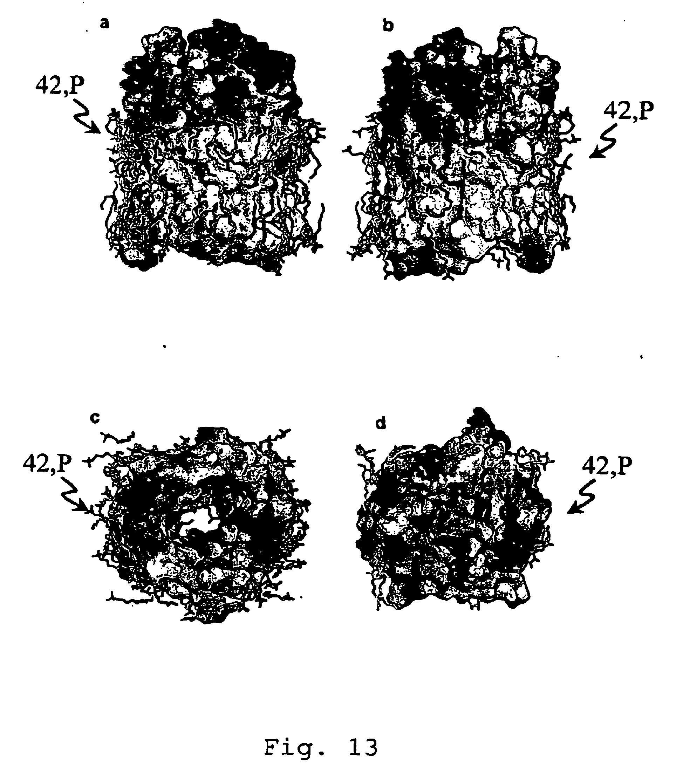

Die Fig. 9A bis 13D zeigen die Tertiär- und Quartärstruktur einer Form des Außenmembranporins G aus Escherichia Coli jeweils in geöffnetem und geschlossenem Zustand sowie die an den offenen und geschlossenen Zuständen der jeweiligen Poren 43 beteiligten Gruppen.

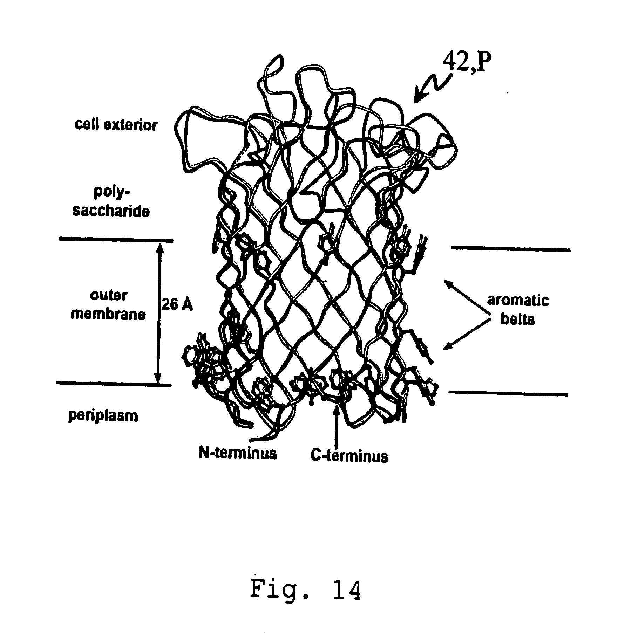

Fig. 14 zeigt in schematischer und geschnittener Seitenansicht, wie in der nativen Umgebung das Außenmembranportn G In Escherichia Coli in die Plasmamembran eingebaut ist.

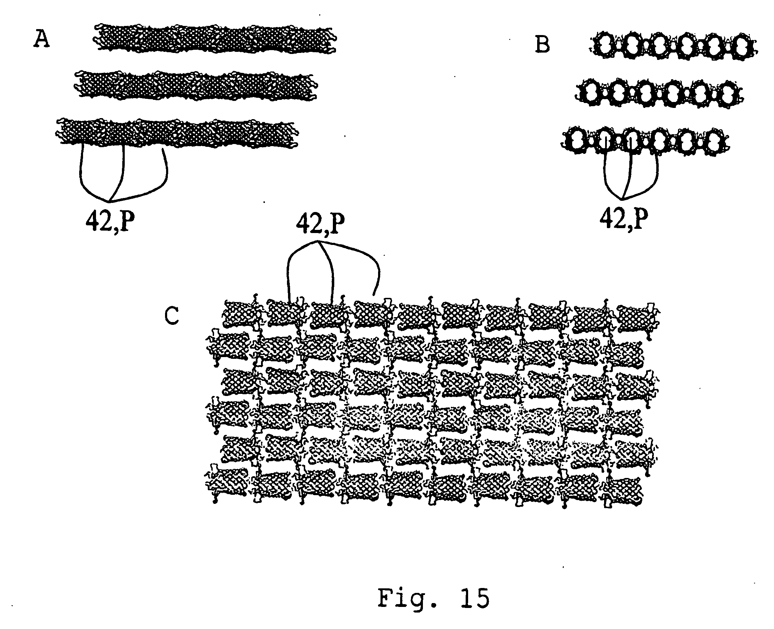

Die Fig. 15A bis 15C zeigen schließlich, dass sich das Außenmembranporin G aus Escherichia Coli aufreinigen und kristallisieren lässt. Aufgrund der kristallinen Packung lässt sich dann eine Röntgenstrukturanalyse durchführen, die dann zu den räumlich aufgelösten Darstellungen gemäß den Fig. 9A bis 13D führt.

### Zitierte Literatur

[1] Duvic. M., Cather. J.. Malze, J. and Frankel. A.E. (1998) DAH(389)IL2 diphtheria fusion toxin produces clinical responses in tumor stage cutaneous T cell lymphoma. American Journal of Hematology, 58. 87-90.
[2] LeMaistre. C.F., Saleh, M.N., Kuzel, T.M., Foss, F., Platanias, L.C., Schwartz, G., Ratain, M., Rook, A., Freytes, C.O., Craig, F., Reuben, J. and Nichols, J.C. (1998) Phase I trial of a ligand fusion-protein (DAB(389)IL-2) in lymphomas expressing the receptor for interleukin-2. Blood. 91. 399-405.

### Bezugezeichenliste

- 1: erfindungsgemäße Sensoranordnung
- 2: erfindungsgemäße Filteranordnung
- 10: erste Elektrodeneinrichtung
- 10a: Oberseite. Oberfläche
- 20: zweite Elektrodeneinrichtung. Gegenelektrode
- 30: wässriges Messmedium
- 31: erstes Kompartiment
- 32: zweites Kompartiment
- 40: funktionelle Membran, biologisch funktionelle Membran
- 40a: Vorderseite
- 40b: Rückseite
- 41: Lipiddoppelschichtmembran. Lipidbilayer
- 41a: Vorderseite
- 41b: Rückseite
- 42: Transmembranprotein, Außenmembranporin G oder OmpG aus Escherichia coli
- 42B: Bindungsstelle
- 42L: Loop, Verschlussmechanismus
- 43: Pore der funktionellen Membran 40
- 50: Messkreis
- 60: Stützkörper, Milliporefilter
- 60a: Oberseite
- 60b: Rückseite
- 61: Pore des Stützkörpers 60
- 100: Gehäuse der Sensoranordnung 1
- 200: Gehäuse der Filteranordnung 2
- 200a: stromaufwärts gelegener Bereich
- 200b: stromabwärts gelegener Bereich
- 210: Septum

- P: Porineinheit, Porinmolekül

## Patentansprüche

1. Anordnung für eine biologisch funktionelle Membran (40),
die eine Lipiddoppelschichtmembran (41) aufweist,
- bei welcher eine Mehrzahl Porineinheiten (P) und/oder Derivate von Porineinheiten (P) membrandurchspannend und funktionell als Poren (42) oder als Kanäle ausgebildet ist.
- bei welcher als Porineinheit (P) ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bet welcher bei der Porineinheit (P) die Reste His231 und His261 jeweils durch Aspartat oder durch Glutamat ersetzt sind.
- um eine Pore (43) der Porineinheit (P) bei neutralem pH-Wert eines Umgebungsmediums (30) zu schließen und bei einem pH-Wert eines Umgebungsmediums (30) unter 4 zu öffnen.

2. Anordnung für eine biologisch funktionelle Membran (40),
die eine Lipiddoppelschichtmembran (41) aufweist,
- bei welcher eine Mehrzahl Porineinheiten (P) und/oder Derivate von Porineinheiten (P) membrandurchspannend und funktionell als Poren (42) oder als Kanäle ausgebildet ist.
- bei welcher als Porineinheit (P) ein Außenmembranporin G aus dem Bakterium Escherichia coli, ein Derivat eines Außenmembranporins G aus dem Bakterium Escherichia coli, ein zum Außenmembranporin G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher bei der Porineinheit (P)
(a) der Rest His231 jeweils durch Aspartat oder durch Glutamat und der Rest His261 jeweils durch Arginin oder Lysin ersetzt sind oder umgekehrt oder
(b) der Rest His261 jeweils durch Aspartat oder durch Glutamat und der Rest His231 jeweils durch Arginin oder Lysin ersetzt sind,
- um eine Pore (43) der Porineinheit (P) in Abhängigkeit von der Ionenstärke eines Umgebungsmediums (30) zu öffnen oder zu schließen.

3. Anordnung für eine biologisch funktionelle Membran (40),
die eine Lipiddoppelschichtmembran (41) aufweist.
- bei welcher eine Mehrzahl Porineinheiten (P) und/oder Derivate von Pordneinheiten (P) membrandurchspannend und funktionell als Poren (42) oder als Kanäle ausgebildet ist.
- brei welcher als Porineinheit (P) ein Außennembranporin G aus dem Bakterium Escherichia coli, ein Derivat eines Außenwembranporins G aus dem Bakterium Escherichia coli, ein zum Außeninembranportn G aus dem Bakterium Escherichia coli oder zu einem Derivat eines Außenmembranporins G aus dem Bakterium Escherichia coli gleichartiges oder homologes 14-strängiges monomeres Porin aus einem Bakterium oder einem Archaebakterium ausgebildet ist, und
- bei welcher bei der Porineinheit (P) die Reste His231 und Hts261 so durch Cystein ersetzt sind.
- dass ein Redoxsensor ausgebildet ist, um eine Pore (43) der Porineinheit (P) unter oxidierenden Bedingungen eines Umgebungsmediums (30) zu öffnen und unter reduzierenden Bedingungen eines Umgebungsmediums (30) zu schließen.

4. Anordnung nach einem der vorangehenden Ansprüche,
bei welcher bei der Porineinheit (P) zwischen dem Anfang der Schleife L6 und dem Ende der Schleife L7 der Aminosäuresequenz der Rest Glu227 und im Bereich der Aminosäuresequenz auf der Innenseite der Pore einer oder mehrere der Reste Phe269. Glu17 und Val19 so durch Cystein ersetzt sind, dass ein Redoxsensor ausgebildet ist.

5. Anordnung nach einem der vorangehenden Ansprüche
- bei weicher die Schleife L6 der Aminosäuresequenz entfernt ist oder
- bei welcher diejenigen Histidinreste der Aminosäuresequenz ersetzt sind, welche zum pH-Sensor der Porineinheit (P) gehören.

6. Anordnung nach einem der vorangehenden Ansprüche,
bei welcher eine Porineinheit (P) vorgesehen ist, die eine spezifische Leitfähigkeit in Abhängigkeit einer Konzentration an Metallionen besitzt, indem die Porineinheit (P) eine Hindungsstelle für Zn²⁺ aufweist:
(a) durch Ersetzen von Glu227 durch Histidin und von einem oder mehreren der Reste Phe269. Glu17 und Val19 jeweils durch Cystein oder
(b) durch Ersetzen von Ile226 und Arg228 durch Histidin und von Thr64 und Phe66 durch Cystein.

7. Anordnung nach einem der vorangehenden Ansprüche.
- bei welcher die funktionelle Membran (40) eine Porineinheit (P) aufweist, die eine spezifische Durchlässigkeit für kleine Moleküle besitzt, wobei die Spezifttät durch hydrophobe, hydrophile, polare, unipolare, aromatische, aliphatische. Ionische und/oder nicht ionische Eigenschaften der Porineinheit (P) und/oder durch die Größe der Moleküle ausgebildet ist.
- bei welcher ein Stützkörper (60) ausgebildet ist, der eine Vorderseite (60a), eine Rückseite (60b) und Poren (61) aufweist, die ihrerseits von der Vorderseite (60a) zur Rückseite (60b) reichen und diese miteinander verbinden.
- bei welcher die Poren (61) durch eine oder durch eine Mehrzahl funktioneller Membranen (40) querschnittsmäßig abgedeckt, überspannt oder verschlossen sind, insbesondere durch jeweils genau eine funktionelle Membran (40), bei welcher der stützkörper (60) mit oder aus einem Material aus der Gruppe ausgebildet ist, die besteht aus anorganischen Materialien, keramischen Materialien und organischen Materialien, und
- bei welcher
(a) der Stützkörpers (60) als Milliporefilter ausgebildet ist, oder
(b) die Poren (61) des Stützkörpers (60) einen Durchmesser im Bereich von etwa 0.2 µm bis etwa 25 µm aufweisen, vorzugsweise im Bereich von etwa 0.2 µm bis etwa 2 µm.

8. Anordnung nach einem der vorangehenden Ansprüche,
- bei welcher eine erste elektrisch leitfähige Elektrodeneinrichtung (10) ausgebildet ist,
- bei welcher eine zweite elektrisch leitfähige Elektrodeneinrichtung (20) ausgebildet ist, und
- bei welcher ein wässriges Messmedium (30. 31. 32), in welchem die erste und die zweite Elektrodeneinrichtung 10, 20) voneinander räumlich getrennt angeordnet sind, ausgebildet ist.
- bei welcher durch die funktionelle Membran (40) die ersten und zweiten Elektrodeneinrichtungen (10, 20) im Bereich des wässrigen Messmediums (30, 31, 32) steuerbar elektrisch voneinander isolierbar und steuerbar elektrisch miteinander verbindbar sind.
- bei welcher durch die funktionelle Membran (40) das Messmedium (30) in ein erstes Kompartiment (31) und In ein zweites und davon getrenntes Kompartiment (32) unterteilt ist,
- bei welcher im ersten Kompartiment (31) die erste Elektrodeneinrichtung (10) zumindest zum Teil angeordnet ist.
- bei welcher im zweiten Kompartiment (32) die zweite Elektrodeneinrichtung (20) zumindest zum Teil angeordnet ist und als Gegenelektrode fungiert,
- bei welcher die funktionelle Membran (40) zumindest zum Teil mit einer Seite (40a) zumindest an einem Teil der Oberfläche (10a) der ersten Elektrodeneinrichtung (10) Immobilisiert ist.
- bei welcher die Immobilisierung der funktionellen Membran (40) an der ersten Elektrodeneinrichtung (10) über eine chemische Bindung ausgebildet ist
- bei welcher die Immobilisierung der funktionellen Membran (40) an der ersten Elektrodeneinrichtung (10) über in der Lipiddoppelschichtmembran (41) vorgesehene funktionelle Gruppen (43), insbesondere über Thiogruppen vorgesehener Thiolipide oder Thioalkane ausgebildet ist.
- bei welcher die erste Elektrodeneinrichtung (10) mit oder aus einem Edelmetall gebildet ist.
- bei welcher das Edelmetall ein Metall aus der Gruppe ist, die gebildet wird von Gold. Silber und Platin und
- bei welcher die erste Elektrodeneinrichtung (10) und die zweite Elektrodeneinrichtung (20) außerhalb des wässrigen Messmediums (30) mit einem Messkreis (50) verbunden sind.

9. Verwendung einer Anordnung nach einem der vorangehenden Ansprüche.
- in einer oder als eine Sensoranordnung (1), insbesondere in einer Biosensoranordnung, in einer oder als eine Filteranordnung (2), als eine Anordnung einer selektiven Elektrode oder für eine selektive Elektrode, oder in einer oder als eine Anordnung eines materiellen Nanofilters oder für einen materiellen Nanofilter, also insbesondere in einem materiellen Filter mit einer Ausschlussgröße, im Bereich bis etwa 2 nm.
- in einem Erfahren zur Herstellung eines Mittels zur In-vitro- oder In-vivo-Ausbildung einer oder mehrerer selektiver oder selektiv gesteuerter oder steuerbarer Permeabilitäten - insbesondere spezifisch - in Membranen eines oder mehrerer Zelitypen, insbesondere im menschlichen oder tierischen Organismus, oder
- in einem Verfahren zur Herstellung eines Mittels zur Krebstherapie,
wobei die funktionelle Membran (40) ausgebildet wird
(a) über zielgerichtete Mutagenese oder in einem Expressionaverfahren.
(b) über einen Obernächenkontakt der Porineinheiten (P) mit einer bestehenden Membran,
(c) über einen Membranfusionsprozess, oder
(d) über einen spezifischen Antikörper-Antigen-Kopplungs- und -Erkennungsprozess.

## Claims

1. Device for a biologically functionalized membrane (40), featuring a lipid bilayer membrane (41),
- in which the majority of porin units (P) and/or derivatives of porin units (P) span the membrane and function as pores (42) or channels,
- in which the porin unit (P) is an outer membrane porin G from the bacterium *Escherichia coli,* a derivative of an outer membrane porin G from the bacterium *Escherichia coli,* or a 14-stranded monomeric porin similar or homologous to an outer membrane porin G from the bacterium *Escherichia coli* or from another bacterium or an archaebacterium, and
- in which residues His231 and His261 of the porin unit (P) are respectively replaced by aspartate or by glutamate
- so as to close a pore (43) of the porin unit (P) when the pH of a surrounding medium (30) is neutral, and to open it when the pH of a surrounding medium (30) is below 4.

2. Device for a biologically functionalized membrane (40), featuring a lipid bilayer membrane (41),
- in which the majority of porin units (P) and/or derivatives of porin units (P) span the membrane and function as pores (42) or channels,
- in which the porin unit (P) is an outer membrane porin G from the bacterium *Escherichia coli,* a derivative of an outer membrane porin G from the bacterium *Escherichia coli,* or a 14-stranded monomeric porin similar or homologous to an outer membrane porin G from the bacterium *Escherichia coli* or from another bacterium or an archaebacterium, and
- in which, in the porin unit (P)
(a) the residue His231 is respectively replaced by aspartate or by glutamate and the residue His261 is replaced by arginine or lysine, or vice versa or,
(b) the residue His261 is respectively replaced by aspartate or by glutamate and the residue His231 is replaced by arginine or lysine,
- in order to open or close a pore (43) of the porin unit (P), depending on the ionic strength of a surrounding medium (30).

3. Device for a biologically functionalized membrane (40), featuring a lipid bilayer membrane (41),
- in which the majority of porin units (P) and/or derivatives of porin units (P) span the membrane and function as pores (42) or channels,
- in which the porin unit (P) is an outer membrane porin G from the bacterium *Escherichia coli,* a derivative of an outer membrane porin G from the bacterium *Escherichia coli,* or a 14-stranded monomeric porin similar or homologous to an outer membrane porin G from the bacterium *Escherichia coli* or from another bacterium or an archaebacterium, and
- in which (P) residues His231 and His261 of the porin unit are replaced by cysteine
- in such a way as to create a redox sensor to open a pore (43) of the porin unit (P) under oxidizing conditions of a surrounding medium (30), and to close it under reducing conditions of a surrounding medium (30).

4. Device according to one of the aforementioned claims,
- in which in the porin unit (P) the residue Glu227 between the start of loop L6 and the end of loop L7 of the amino acid sequence, and one or several of the residues Phe269, Glu17 and Val19 in the region of the amino acid sequence on the inside of the pore are replaced by cysteine, such that a redox sensor is created.

5. Device according to one of the aforementioned claims,
- in which loop L6 of the amino acid sequence is removed or
- in which those histidine residues in the amino acid sequence are replaced that are part of the pH sensor of the porin unit (P).

6. Device according to one of the aforementioned claims,
- in which a porin unit (P) is intended to have a specific conductivity in response to a concentration of metal ions, where the pore unit (P) features a binding site for Zn²+:
(a) by replacing Glu227 with histidine and by replacing one or several of the residues Phe269, Glu17 and Val19 each by cysteine or
(b) by replacing Ile226 and Arg228 by histidine and Thr64 and Phe66 by cysteine.

7. Device according to one of the aforementioned claims,
- in which the functionalized membrane (40) features a porin unit (P) with a specific permeability to small molecules, where the specificity is defined by the hydrophobic, hydrophilic, polar, unipolar, aromatic, aliphatic, ionic and/or non-ionic qualities of the porin unit (P) and/or the size of the molecules,
- which features a supporting body (60) comprising a front side (60a), a reverse side (60b), and pores (61), that reach from the front side (60a) to the reverse side (60b) and connect both sides,
- in which the pores (61) are covered by one or a multitude of functionalized membranes (40) stretching across or closing them, in particular by exactly one functionalized membrane (40),
- in which the supporting body (60) is formed with or consists of a material from a group consisting of inorganic materials, ceramic materials and organic materials and
- in which
(a) the supporting body (60) is a Millipore filter, or
(b) the pores (61) of the supporting body (60) have a diameter from approximately 0.2µm to approximately 25µm, preferentially in the range of approximately 0.2µm to approximately 2 µm.

8. Device according to one of the aforementioned claims
- which features a first electrically conducting electrode device (10)
- which features a second electrically conducting electrode device (20) and
- which features an aqueous medium (30,31, 32), in which the first and the second electrode device (10, 20) are arranged such that they are physically separated,
- in which the functionalized membrane (40) electrically can isolate the first and the second electrode devices (10, 20) within the aqueous measuring medium (30, 31, 32) from one another in a controlled manner, and can electrically connect them to one another in a controlled manner (40),
- in which the functionalized membrane (40) divides the measuring medium (30) into a first compartment (31) and a separate second compartment (32),
- in which at least part of the first electrode device (10) is arranged in the first compartment (31),
- in which at least part of the second electrode device (20) is arranged in the second compartment (32), and functions as a counter electrode,
- in which the functionalized membrane (40) is immobilized at least partly on one side (40a) on at least part of the surface (10a) of the first electrode device (10),
- in which the functionalized membrane (40) is immobilized on the first electrode device (10) by a chemical bond,
- in which the functionalized membrane (40) is immobilized on the first electrode device (10) by functional groups (43) intended for this purpose in the lipid bilayer membrane (41), in particular through thio-groups of thio-lipids or thio-alkanes intended for this purpose,
- in which the first electrode device (10) is formed with or consists of a noble metal,
- in which the noble metal is from a group consisting of gold, silver and platinum and
- in which the first electrode device (10) and the second electrode device (20) are connected outside the aqueous measuring medium (30) in a measuring circuit (50).

9. Application of a device according to the aforementioned claims,
- in or as a sensory device (1), in particular as a biosensor device, in or as a filter device (2), as a device of a selective electrode, or for a selective electrode, or in or as a device of a material nanofilter or for a material nanofilter, in particular as a material filter with an exclusion size in the range of approximately 2 nm,
- in a procedure for producing an agent for one or several selective or selectively controlled or controllable permeabilities in vitro or in vivo - in particular and specifically - in membranes of one or several cell types, in particular in the human body or in animals, or
- in a procedure for producing an agent for cancer therapy,
where the functionalized membrane (40) is created
(a) by targeted mutagenesis or through an expression procedure,
(b) by a surface contact of the porin unit (P) to an existing membrane,
(c) by a process of membrane fusion, or
(d) through a process of specific antibody-antigen coupling and recognition.

## Revendications

1. Agencement pour une membrane biofonctionnelle (40) qui présente une membrane lipidique à deux couches (41),
dans lequel une pluralité de motifs de porine (P) et/ou de dérivés de motifs de porine (P) est constituée de façon à traverser la membrane et à former fonctionnellement des pores (42) ou des canaux,
dans lequel, comme motif de porine (P), une porine de membrane externe G issue de la bactérie *Escherichia coli,* un dérivé d'une porine de membrane externe G de la bactérie *Escherichia coli,* une porine monomère à 14 brins de même type ou homologue à la porine de membrane externe G de la bactérie *Escherichia coli* ou d'un dérivé d'une porine de membrane externe G de la bactérie *Escherichia coli* est formée à partir d'une bactérie ou d'une archéobactérie et
dans lequel les radicaux His231 et His261 du motif de porine (P) sont remplacés respectivement par l'aspartate ou le glutamate,
pour fermer un pore (43) du motif de porine (P) à un pH neutre d'un milieu environnemental (30) et pour l'ouvrir à un pH d'un milieu environnemental (30) inférieur à 4.

2. Agencement destiné à une membrane biofonctionnelle (40) qui présente une membrane lipidique à deux couches (41)
dans lequel une pluralité de motifs de porine (P) et/ou de dérivés de motifs de porine (P) est constituée de façon à traverser la membrane et à former fonctionnellement des pores (42) ou des canaux,
dans lequel, comme motif de porine (P) une porine de membrane externe G issue de la bactérie *Escherichia coli,* un dérivé d'une porine de membrane externe G de la bactérie *Escherichia coli,* une porine monomère à 14 brins de même type ou homologue à la porine de membrane externe G de la bactérie *Escherichia coli* ou d'un dérivé d'une porine de membrane externe G de bactérie *Escherichia coli* est formée à partir d'une bactérie ou d'une archéobactérie et
dans lequel, dans le motif de porine (P)
(a) le radical His231 est remplacé respectivement par un aspartate ou par un glutamate et le radical His261 est remplacé respectivement par une arginine ou une lysine ou inversement, ou
(b) le radical His261 est remplacé respectivement par un aspartate ou par un glutamate et le radical His231 est remplacé respectivement par une arginine ou une lysine, pour ouvrir ou fermer un pore (43) du motif de porine (P) en fonction des forces ioniques d'un milieu environnemental (30).

3. Agencement destiné à une membrane biofonctionnelle (40) qui présente une membrane lipidique à deux couches (41),
dans lequel une pluralité de motifs de porine (P) et/ou de dérivés de motifs de porine (P) est constituée de façon à traverser la membrane et à former fonctionnellement des pores (42) ou des canaux,
dans lequel, comme motif de porine (P) une porine de membrane externe G issue de la bactérie *Escherichia coli,* un dérivé d'une porine de membrane externe G de la bactérie *Escherichia coli,* une porine monomère à 14 brins de même type ou homologue à la porine de membrane externe G de la bactérie *Escherichia coli* ou d'un dérivé d'une porine de membrane externe G de bactérie *Escherichia coli* est formée à partir d'une bactérie ou d'une archéobactérie et
dans lequel les radicaux His231 et His261 du motif de porine (P) sont remplacés par la cystéine,
un capteur redox est formé pour ouvrir un pore (43) du motif de porine (P) dans des conditions oxydantes d'un milieu environnemental (30) et pour le fermer dans des conditions réductrices d'un milieu environnemental (30).

4. Agencement selon l'une des revendications précédentes, dans lequel dans l'unité de porine (P) entre le début de la boucle L6 et la fin de la boucle L7 de la séquence d'acides aminés, le radical Glu227 et dans la région de la séquence d'acides aminés sur la face interne des pores, un ou plusieurs des radicaux Phe269, Glu17 et Val19 sont remplacés par une cystéine et un capteur rédox est formé.

5. Agencement selon l'une des revendications précédentes,
dans lequel la boucle L6 de la séquence d'acides aminés est éloignée ou
dans lequel les radicaux histidine de la séquence d'acide aminé qui font partie du capteur de pH du motif de porine sont remplacés.

6. Agencement selon l'une des revendications précédentes,
dans lequel une unité de porine (P) est prévue, qui possède une capacité de conduction spécifique en fonction d'une concentration d'ions métalliques (1), le motif de porine (P) présentant un site de liaison de Zn²⁺ :
(a) par le remplacement de Glu227 par l'histidine et d'un ou plusieurs des radicaux Phe269, Glu17 et Val19, respectivement par une cystéine ou
(b) par le remplacement de Il226 et Arg228 par une histidine et de Thr64 et Phe66 par une cystéine.

7. Agencement selon l'une des revendications précédentes,
dans lequel la membrane fonctionnelle (40) présente un motif de porine (P) qui possède une perméabilité spécifique aux petites molécules, la spécificité étant constituée par des propriétés hydrophobes, hydrophiles, polaires, unipolaires, aromatiques, aliphatiques, ioniques et/ou non ioniques du motif de porine (P) et/ou par la taille des molécules,
dans lequel un corps de soutien (60) est constitué, qui présente une face avant (60a), une face arrière (60b) et des pores (6) qui vont eux-mêmes de la face avant (60a) à la face arrière (60b) et relient celles-ci,
dans lequel les pores (61) sont recouverts perpendiculairement, compris ou renfermés par une ou plusieurs des membranes fonctionnelles (40), en particulier par respectivement exactement une membrane fonctionnelle (40),
dans lequel le corps d'appui (60) est formé avec ou d'un matériau du groupe comprenant les matériaux anorganiques, les matériaux céramiques et les matériaux organiques, et
dans lequel
(a) le corps d'appui (60) est constitué comme un filtre millipore, ou
(b) les pores (61) du corps d'appui (60) présentent un diamètre d'environ 0,2 µm à environ 25 µm, de préférence d'environ 0,2 µm à environ 2 µm.

8. Agencement selon l'une des revendications précédentes,
dans lequel un premier dispositif à électrodes électriquement conducteur (10) est formé,
dans lequel un deuxième dispositif à électrodes (20) électriquement conducteur est formé et
dans lequel un milieu de mesure aqueux (30, 31, 32) dans lequel les premier et deuxième dispositifs à électrodes (10, 20) sont séparés spatialement l'un de l'autre, est formé
dans lequel par la membrane fonctionnelle (40), les premier et deuxième dispositifs à électrodes (10, 20) peuvent être isolés et reliés électriquement l'un de l'autre dans la zone du milieu de mesure aqueux (30, 31, 32) de façon commandable,
dans lequel dans la membrane fonctionnelle (40), le milieu de mesure (30) est subdivisé en un compartiment (31) et en un deuxième compartiment (32) séparé de celui-ci,
dans lequel, le premier dispositif à électrodes (10) est disposé au moins en partie dans le premier compartiment (31),
dans lequel, le deuxième dispositif à électrodes (20) est disposé au moins en partie dans le deuxième compartiment (32) et fait office de contre-électrode,
dans lequel la membrane fonctionnelle (40) est immobilisée au moins en partie avec une face (40a) au moins sur une partie de la surface (10a) du premier dispositif à électrodes (10),
dans lequel l'immobilisation de la membrane fonctionnelle (40) sur le premier dispositif à électrodes (10) est obtenue par une liaison chimique,
dans lequel l'immobilisation de la membrane fonctionnelle (40) sur le premier dispositif à électrodes (10) est obtenue par des groupes fonctionnels (43) prévus dans la membrane lipidique à double couche, en particulier par les groupes thio des lipides thiol prévus,
dans lequel le premier dispositif à électrodes (10) est formé avec un, ou en, métal noble,
dans lequel le métal noble est un métal du groupe constitué par l'or, l'argent et le platine, et
dans lequel le premier dispositif à électrodes (10) et le deuxième dispositif à électrode (20) son reliés à l'extérieur du milieu de mesure aqueux (30) avec un circuit de mesure (50).

9. Utilisation d'un agencement selon l'une des revendications précédentes,
dans ou comme dispositif de capteur (1), en particulier dans un agencement de biocapteur, dans ou comme agencement de filtrage (2), comme agencement d'une électrode sélective ou pour une électrode sélective ou dans ou comme agencement d'un nanofiltre physique ou pour un nanofiltre physique, donc en particulier dans un nanofiltre physique présentant une taille d'exclusion dans une plage allant jusqu'à environ 2 nm,
dans un procédé de production d'un moyen de formation *in vitro* ou *in vivo* d'une ou plusieurs perméabilités sélectives ou commandées sélectivement ou pouvant être commandées sélectivement, en particulier spécifiquement dans des membranes d'un ou plusieurs types cellulaires, en particulier dans un organisme humain ou animal, ou
dans un procédé de production d'un agent destiné au traitement du cancer,
la membrane fonctionnelle (40) étant constituée
(a) par mutagenèse ciblée ou dans un procédé d'expression
(b) par un contact de surface des motifs de porine (P) avec une membrane existante,
(c) par un processus de fusion membranaire ou
(d) par un processus de couplage et d'identification d'un anticorps-antigène spécifique.
